# EUROPÄISCHE PATENTSCHRIFT

(11) **EP 0 467 206 B1**
(45) Veröffentlichungstag und Bekanntmachung des Hinweises auf die Patenterteilung: **18.12.1996**
(21) Anmeldenummer: 91111462.7
(22) Anmeldetag: 10.07.1991
(51) Int. Cl.: C07D 495/04, C07D 491/04, A01N 43/90

(54) **Dicarbonsäureimide, Verfahren zu ihrer Herstellung und ihre Verwendung als Herbizide**
Dicarboxylic acid imides, processes for their preparation and their use as herbicides
Imides d'acides dicarboxyliques, leurs procédés de préparation et leur utilisation à titre d'herbicides

(30) Priorität: 20.07.1990 DE 4023048
(43) Veröffentlichungstag der Anmeldung: 22.01.1992
(73) Patentinhaber: BASF Aktiengesellschaft, 67063 Ludwigshafen (DE)
(72) Erfinder: Muenster, Peter, Dr., W-6823 Neulussheim (DE); Freund, Wolfgang, Dr., W-6730 Neustadt (DE); Steiner, Gerd, Dr., W-6719 Kirchheim (DE); Walter, Helmut, Dr., W-6719 Obrigheim (DE); Westphalen, Karl-Otto, Dr., W-6720 Speyer (DE); Gerber, Matthias, Dr., W-6704 Mutterstadt (DE)

(56) Entgegenhaltungen:
- EP-A- 0 259 264
- EP-A- 0 423 523
- DE-A- 2 538 951
- DE-A- 3 402 026
- DE-A- 3 901 074

## Beschreibung

Die vorliegende Erfindung betrifft Dicarbonsäureimide der allgemeinen Formeln Ia und Ib, 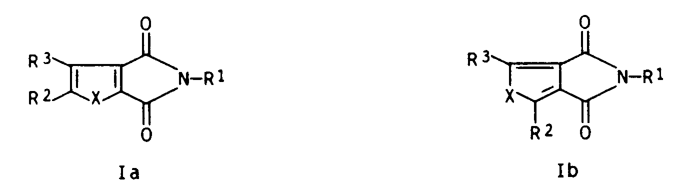 in denen die Substituenten folgende Bedeutung haben:
X
   Sauerstoff oder Schwefel;
R¹
   Wasserstoff, Hydroxy,
   C₃-C₈-Cycloalkyl, welches ein bis drei der folgenden Reste tragen kann: Halogen, C₁-C₄-Alkyl, C₁-C₄-Halogenalkyl, C₁-C₄-Alkoxy und/oder C₁-C₄-Halogenalkoxy;
   C₁-C₆-Alkyl, welches ein bis drei der folgenden Reste tragen kann: Hydroxy, Halogen, Cyano, C₃-C₈-Cycloalkyl, C₁-C₄-Alkoxy, C₁-C₄-Halogenalkoxy, C₁-C₄-Alkylthio, C₁-C₄-Halogenalkylthio, C₁-C₄-Alkylamino, Di-C₁-C₄-alkylamino und/oder C₃-C₆-Cycloalkylamino und/oder einen Rest 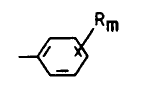 tragen kann, wobei
R
   Cyano, Nitro, Halogen, C₁-C₄-Alkyl, C₁-C₄-Halogenalkyl, C₁-C₄-Alkoxy, C₁-C₄-Halogenalkoxy, C₂-C₄-Alkinyloxy, C₁-C₄-Alkylthio, C₁-C₄-Halogenalkylthio, C₃-C₆-Alkoxycarbonylalkoxy, C₁-C₄-Alkoxycarbonyl, 2-Alkoxycarbonylprop-1-enyl, C₁-C₄-Alkanoyl, C₁-C₄-Halogenalkanoyl, Formyl, Dioxolanyl und/oder Phenyl bedeutet und
m
   für 0, 1, 2 oder 3 steht, wobei die Reste R verschieden sein können, wenn m 2 oder 3 bedeutet;
   C₁-C₄-Alkoxy, C₂-C₆-Cyanalkyl, C₃-C₆-Alkenyl, C₃-C₆-Alkinyl, Phenyl oder Naphthyl, wobei diese Gruppen ein bis drei der bei R genannten Reste tragen können;
   einen 5- bis 6-gliedrigen gesättigten oder ungesättigten Heterocyclus, enthaltend ein oder zwei Heteroatome ausgewählt aus der Gruppe Stickstoff, Sauerstoff und Schwefel, wobei dieser Ring ein oder zwei der folgenden Reste tragen kann: Halogen, C₁-C₄-Alkyl, C₁-C₄-Halogenalkyl, C₁-C₄-Alkoxy, C₁-C₄-Halogenalkoxy und/oder C₁-C₄-Alkylthio,
   Di-C₁-C₄-alkylamino;
R², R³
   Nitro, Cyano, Halogen,
   Amino, welches ein oder zwei C₁-C₄-Alkylgruppen und/oder eine C₁-C₄-Alkylcarbonylgruppe tragen kann;
   C₁-C₄-Alkoxy oder C₁-C₄-Alkylthio, wobei diese Gruppen ein bis neun Halogenatome tragen können;
   C₁-C₄-Alkylsulfonyl, C₁-C₄-Halogenalkylsulfonyl,
   C₂-C₆-Alkenyl, C₂-C₆-Alkinyl, Phenyl, Phenoxy oder Phenylthio, wobei diese Gruppen ein bis drei der bei R genannten Reste tragen können, oder
   eine der bei R¹ genannten Gruppen;
   mit der Maßgabe, daß im Falle des Dicarbonsäureimids Ia mit X = Schwefel
   - R¹ nicht Wasserstoff und 2-Methyl-4-nitro-phenyl bedeutet, wenn R² und R³ für Wasserstoff stehen;
   - R² nicht Amino bedeutet, wenn R³ für Cyano steht;
   ferner mit der Maßgabe, daß im Fall des Dicarbonsäureimids Ib mit
X = Sauerstoff
   - R¹ nicht Phenyl oder Wasserstoff bedeutet, wenn R² und R³ für Phenyl stehen;
   - nicht alle Reste R¹ - R³ gleichzeitig Methyl bedeuten;
   sowie schließlich mit der Maßgabe, daß im Fall der Dicarbonsäureimide Ib mit X = Schwefel
   - R¹ nicht Phenyl bedeutet, wenn R² und R³ für Wasserstoff stehen;
   - nicht alle Reste R¹ - R³ gleichzeitig Wasserstoff bedeuten;
   - R² und R³ nicht gleichzeitig für Phenyl, das ein Rest Chlor, Brom, Cyano, Alkoxycarbonyl oder Alkoxy tragen kann, stehen;
   sowie landwirtschaftlich brauchbare Salze der Verbindungen Ia oder Ib.

Außerdem betrifft die Erfindung ein Verfahren zur Herstellung der Verbindungen Ia, Ib und herbizide Mittel, enthaltend mindestens ein Dicarbonsäureimid Ia, Ib und/oder ein Dicarbonsäureimid der allgemeinen Formeln IA und IB 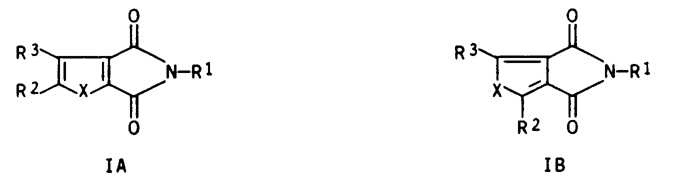
in denen die Substituenten die in Anspruch 1 gegebene Bedeutung haben und zusätzlich die dort ausgeschlossenen Verbindungen vorliegen können.

Formel Ia mit X = Schwefel sind bekannt aus DE-A 25 09 922, J. Chem. Soc. 1937, S. 911 sowie DE-A 34 02 026 oder Synthesis 1988, 499 ff. Dicarbonsäureimide der Formel Ib mit X = Sauerstoff sind bekannt aus Chem. Ber. 58, 1783-1787 (1925) und Chem. Commun. 22, 1466-67 (1984). Dicarbonsäureimide der Formel Ib mit X = Schwefel sind bekannt aus Chem. Ber. 111, 3029-3036 (1978), DE-A 25 38 951, J. Org. Chem. 19, S. 70 (1954) und Egypt. J. Chem. 24, S. 371-373 (1981). Dem zitierten Stand der Technik sind keine Hinweise auf herbizide Eigenschaften der Dicarbonsäureimide zu entnehmen.

Der vorliegenden Erfindung lagen neue herbizid wirksame Substanzen als Aufgabe zugrunde.

Demgemäß wurden die eingangs definierten Dicarbonsäureimide Ia und Ib und Verfahren zu ihrer Herstellung gefunden. Des weiteren wurde gefunden, daß sich sowohl die Dicarbonsäureimide Ia und Ib als auch die Dicarbonsäureimide IA und IB zur Bekämpfung unerwünschten Pflanzenwuchses eignen.

Die erfindungsgemäßen Dicarbonsäureimide Ia und Ib sind auf verschiedenen wegen herstellbar. Man erhält sie beispielsweise nach den folgenden Verfahren.

### Weg A:

Durch Wasserentzug mit wasserabspaltenden Mitteln, beispielsweise Acetanhydrid oder anorganischen Säurehalogeniden, werden die Verbindungen II und III in die Dicarbonsäureimide der Formel Ia sowie die Verbindungen IV und V in die Dicarbonsäureimide der Formel Ib umgewandelt. Die Reaktion wird zweckmäßigerweise so durchgeführt, daß man die Carbonsäureamide in einem inerten organischen Lösungsmittel vorlegt und etwa molare Mengen eines wasserabspaltenden Mittels, gegebenenfalls ebenfalls gelöst in einem inerten Lösungsmittel, zugetropft. Das Gemisch kann wie üblich aufgearbeitet werden, beispielsweise durch Hydrolyse mit Wasser und Absaugen oder Extraktion des Produktes mit einem organischen Lösungsmittel und Einengen des organischen Lösungsmittels:

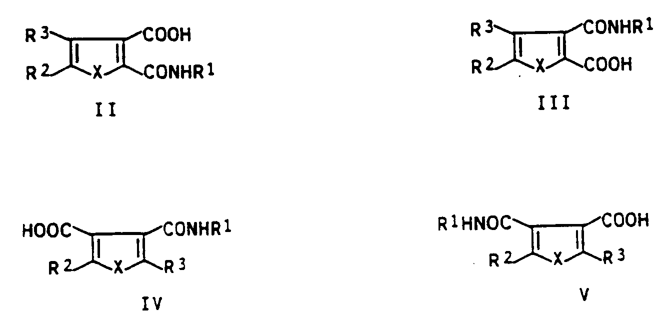

Zweckmäßigerweise verwendet man für diese Umsetzungen Lösungsmittel wie Halogenkohlenstoffe, z.B. Tetrachlorethan, Methylenchlorid, Chloroform, Dichlorethan, Chlorbenzol und 1,2-Dichlorbenzol, Ether, z.B. Diethylether, Methyl-tert.-butylether, Dimethoxyethan, Diethylenglykoldiethylether, Tetrahydrofuran und Dioxan; dipolare aprotische Lösungsmittel, z. B. Acetonitril, Dimethylformamid, Dimethylacetamid, Dimethylsulfoxid, N-Methylpyrrolidon, 1,3-Dimethyltetrahydro-2(1H)-pyrimidinon und 1,3-Dimethylimidazolin-2-on; Aromaten, z. B. Benzol, Toluol, Xylol, Pyridin und Chinolin; Ketone, z. B. Aceton, Methylethylketon oder entsprechende Gemische.

Wasserabspaltende Mittel sind beispielsweise Anhydride niederer Alkansäuren wie Essigsäureanhydrid, Propanphosphonsäureanhydrid, Toluolsulfonsäurechlorid/Pyridin, Thionylchlorid oder Phosphortri- oder pentabromid oder -chlorid.

Die Umsetzung kann bei Temperaturen von -10°C bis zur Rückflußtemperatur des jeweiligen Lösungsmittels, vorzugsweise bei 0 bis 150°C, durchgeführt werden.

Die molaren Verhältnisse, in denen die benötigten Ausgangsverbindungen miteinander umgesetzt werden, betragen im allgemeinen 0,9 : 1 bis 5 : 1 für das Verhältnis von wasserabspaltendem Mittel zu Säureamid.

Die Konzentration der Edukte im Lösungsmittel(gemisch) beträgt im allgemeinen 0,1 bis 5 mol/l, bevorzugt 0,2 bis 2 mol/l.

Die als Ausgangsmaterialien verwendeten Dicarbonsäuremonoamide II bis V können auf verschiedenen Wegen synthetisiert werden.

Die Dicarbonsäuremonoamide II - V können z. B. erhalten werden, indem man eine entsprechende Carbonsäure der Formel IX a - d in an sich bekannter Weise in das Halogenid oder eine andere aktivierte Form der Carbonsäure überführt, diese Derivate anschließend mit einem Amin der Formel VIII umsetzt und das so erhaltene Amid X a-d dann in Gegenwart einer Base mit einem Carboxylierungsreagenz umsetzt.

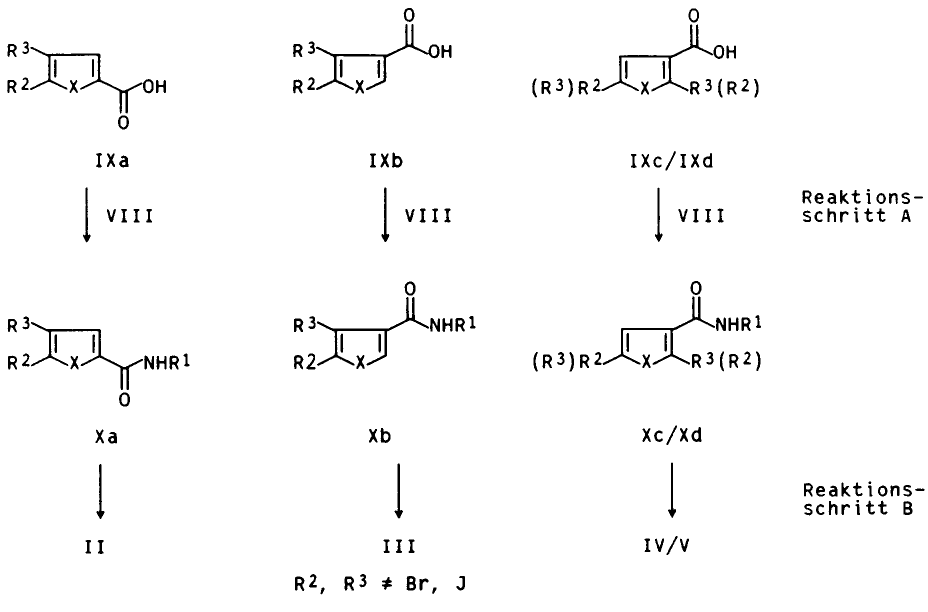

Die einzelnen Reaktionsschritte dieser Synthesesequenz können im allgemeinen wie folgt durchgeführt werden:

### Reaktionsschritt A:

Man erhält die Verbindungen Xa-d aus den Säuren IXa-d, indem man IXa-d zunächst in an sich bekannter Weise in das Halogenid oder eine andere aktivierte Form der Carbonsäurefunktion überführt und diese Derivate anschließend mit einem Amin VIII amidiert.

Aktivierte Formen der Carbonsäure sind neben Halogeniden wie insbesondere den Chloriden und den Bromiden beispielsweise auch Imidazolide. Im allgemeinen werden die Halogenide bevorzugt.

Man erhält sie durch Umsetzung der Carbonsäuren IXa-d mit einem Halogenierungsmittel wie Thionylchlorid, Thionylbromid, Phosphoroxychlorid bzw. -bromid, Phosphortri- und -pentachlorid bzw. -bromid, Phosgen sowie elementarem Chlor und Brom.

Das Halogenierungsmittel wird in 1 bis 5 mol-äq., vorzugsweise 1 bis 2 mol-äq., eingesetzt.

Die Umsetzung verläuft bei Temperaturen von 20°C bis zum Siedepunkt des Halogenierungsmittels bzw. sofern man in Gegenwart eines inerten organischen Lösungsmittels arbeitet, auch dessen Siedepunkt.

Als Lösungsmittel eignen sich beispielsweise Kohlenwasserstoffe und Halogenkohlenwasserstoffe wie Benzol, Toluol und Dichlormethan.

Üblicherweise werden die aktivierten Carbonsäurederivate isoliert, beispielsweise durch Abdestillieren des Halogenierungsmittels und sofern vorhanden des Lösungsmittels und erst anschließend mit den Aminen VIII umgesetzt.

In diesem Fall wird die Amidierung bei Temperaturen von -20 bis 50°C, vorzugsweise 0 bis 30°C in einem inerten aprotisch polaren organischen Lösungsmittel durchgeführt.

Für diese Umsetzung eignen sich insbesondere Halogenkohlenwasserstoffe wie Dichlormethan und Ether wie Diethylether und tert.-Butylmethylether als Lösungsmittel.

Da bei der Amidierung von Säurehalogeniden Halogenwasserstoff gebildet wird, empfiehlt es sich, das Amin VIII in 2 bis 5 mol.-äq. Überschuß, vorzugsweise 2 bis 3 mol.-äq. zuzusetzen. Sofern das Amin in äquimolaren Mengen (1 bis 1,2 mol-äq.) eingesetzt wird, ist es vorteilhaft, zum Binden des Halogenwasserstoffs eine Base, insbesondere ein tertiäres Amin wie Triethylamin oder Pyridin zuzugeben.

### Reaktionsschritt B:

Die Carboxylierung der Carbonsäureamide Xa-d erfolgt in der Regel bei Temperaturen von -100 bis +20°C, vorzugsweise -80 bis -40°C in einem aprotisch polaren inerten organischen Lösungsmittel unter Ausschluß von Feuchtigkeit in Gegenwart einer Base.

Bevorzugtes Carboxylierungsmittel ist Kohlendioxid.

Geeignete Lösungsmittel sind insbesondere Ether wie Diethylether, tert.-Butyl-methylether, Tetrahydrofuran und Dioxan.

Als Basen finden bevorzugt Alkalimetallkohlenwasserstoffe wie Methyllithium, n-Butyllithium, tert.-Butyllithium und Phenyllithium Verwendung.

Die Umsetzung wird üblicherweise so durchgeführt, daß zunächst eine Lösung des Carbonsäureamids (Xa-d) mit 1,3 bis 2,5 mol-äq. der gelösten Base versetzt wird, wobei ein im Ring metallisiertes Carbonsäureamid-derivat entsteht, welches bei der anschließenden Zugabe des elektrophilen Carboxylierungsreagens' zum gewünschten Produkt II-V abreagiert.

Die für dieses Verfahren benötigten Carbonsäuren IXa-d sind literaturbekannt oder können nach allgemeinen literaturbekannten Methoden, z.B. durch Oxidation aus den entsprechenden Alkoholen oder Aldehyden oder durch Hydrolyse aus den entsprechenden Nitrilen hergestellt werden (Beilstein, Hauptwerk und 1. bis 5. Ergänzungswerk, Band 18; The Chemistry of Heterocyclic Compounds, Interscience Publishers, New York, 1976, John Wiley & Sons, Inc., 1988, Vol. 44, Part I-III).

Verfahren zur Herstellung der Verbindungen XI und XII, in denen R² oder R³ Halogen und R' Alkyl bedeutet:

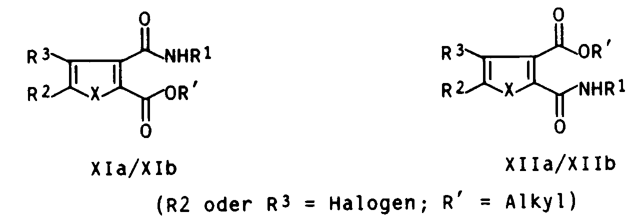

Man erhält diese Verbindungen XI und XII dadurch, daß man einen Dicarbonsäurediester der allgemeinen Formel XIII oder XIV in an sich bekannter Weise zunächst diazotiert und die diazotierte Verbindung mit einem anorganischen Halogenid in das entsprechende Derivat XV oder XVI überführt, anschließend mit einem Amin der Formel VIII amidiert und das so erhaltene Gemisch der isomeren Verbindungen XIa und XIb bzw. XIIa und XIIb in die Einzelkomponenten auftrennt.

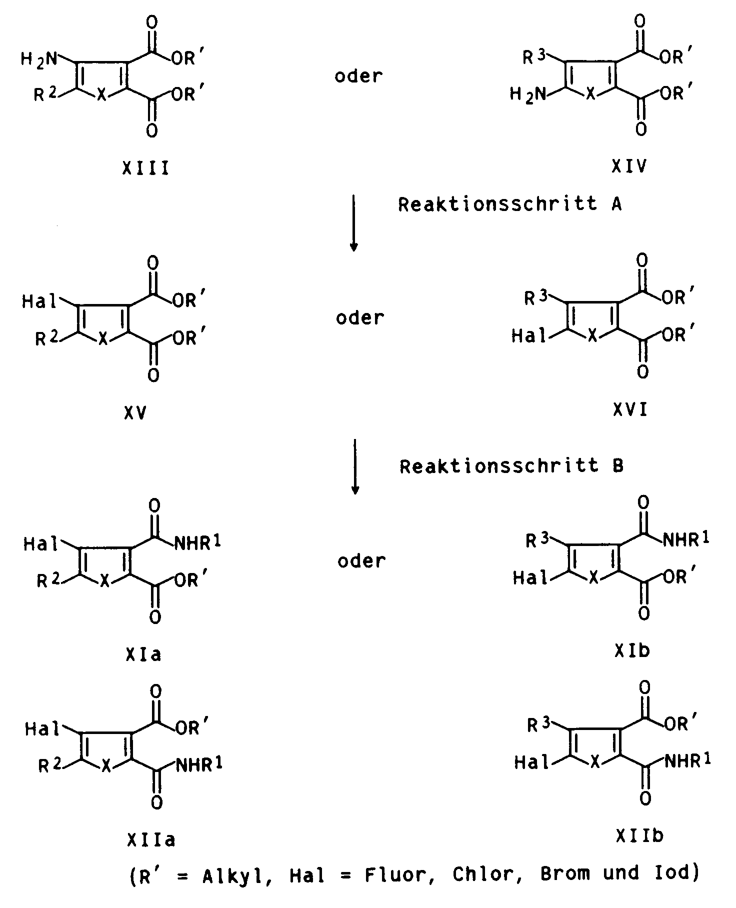

Die Reaktionsschritte dieser Synthesesequenz können im allgemeinen wie folgt durchgeführt werden:

### Reaktionsschritt A:

Die Diazotierung der Dicarbonsäureester der allgemeinen Formel XIII bzw. XIV erfolgt in der Regel bei Temperaturen von -20 bis +20°C, vorzugsweise -5 bis +10°C in einer Mineralsäure wie insbesondere Salzsäure in Gegenwart eines Alkalimetallnitrits wie Natriumnitrit.

Das so erhaltene Diazoniumsalz wird anschließend in situ mit 1 bis 5 mol, vorzugsweise 1,5 bis 2,5 mol eines anorganischen Halogenids insbesondere eines Kupfer(I)halogenids umgesetzt.

Die Reaktionsbedingungen sind im Rahmen der für die Sandmeyer-Reaktion bekannten Verfahren variabel (s.a. Houben-Weyl, Bd. X/3, S. 1-212 (1965); Chem. Zvesti 36, 401 (1982)).

### Reaktionsschritt B:

Die Umsetzung der so erhaltenen Dicarbonsäureester XV bzw. XVI mit dem Amin VIII erfolgt im allgemeinen und im besonderen analog zu den im nachfolgenden Verfahren geschilderten Bedingungen.

Insbesondere finden als Lösungsmittel hierbei jedoch Halogenkohlenwasserstoffe wie Methylenchlorid und Ether wie Diethylether, tert.-Butyl-methylether und Tetrahydrofuran Anwendung.

Das Amin VIII wird im allgemeinen in äquimolaren Mengen oder im Überschuß, vorzugsweise in Mengen von 1 bis 1,2 mol-äq, bezogen auf XV bzw. XVI eingesetzt.

Bei diesem Verfahren entstehen die isomeren Carbonsäureamide der Formeln XIa/b bzw. XIIa/b in unterschiedlichen Mengen. Die Auftrennung des Isomerengemischs gelingt entweder durch fraktionierte Kristallisation oder auf chromatographischem Wege.

Die für dieses Verfahren benötigten Dicarbonsäurediester XIII sind bekannt oder können aus den entsprechenden Oxoestern XVII, beispielsweise analog den in Synthesis, 1977, 200 beschriebenen Bedingungen gemäß dem folgenden Reaktionsschema hergestellt werden:

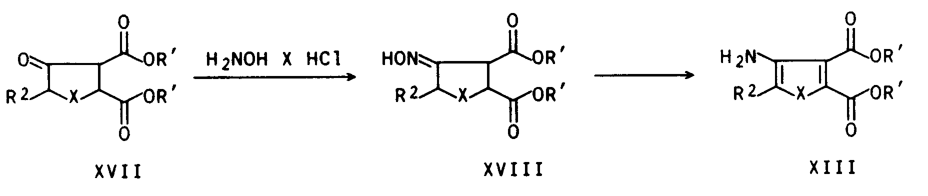

### Verfahren zur Herstellung der Verbindungen II und III

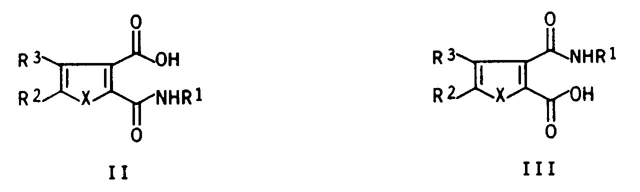

Man erhält diese Carbonsäureamide II bzw. III beispielsweise dadurch, daß man ein entsprechendes Carbonsäureamid der Formel XIa/b bzw. XIIa/b, in der R' Alkyl bedeutet, in an sich bekannter Weise mit einer wäßrigen Base hydrolysiert.

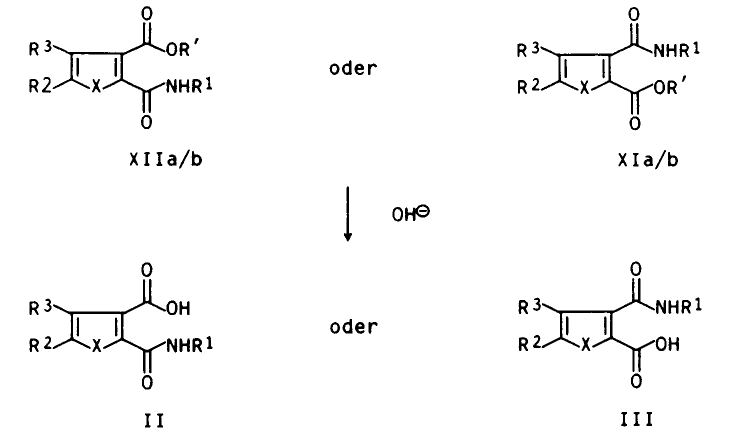

Die Reaktion wird so durchgeführt, daß man ein Carbonsäureamid XIa/b oder XIIa/b (R' = Alkyl) in einem inerten Lösungsmittel vorlegt und bei -30 bis 120°C, vorzugsweise bei -10 bis 40°C, mit einer wäßrigen Base umsetzt. Die Carbonsäureamide der Formel II bzw. III (R' = H) werden dann bei -30 bis 100°C, vorzugsweise bei -10 bis 10°C, durch Zugabe von Mineralsäuren freigesetzt.

Als Lösungsmittel für diese Esterspaltung kommen Alkohole wie Methanol, Ethanol, Propanol oder Ethylenglykol in Betracht; besonders bevorzugt arbeitet man in dem gleichen Alkohol, der der Esterkomponente R'OH entspricht. Die Konzentration des Edukts XVII oder XVIII beträgt im allgemeinen 0,1 bis 5,0 mol/l, bevorzugt 0,2 bis 2,0 mol/l.

Als wäßrige Base setzt man wäßrige Lösungen von Alkali- oder Erdalkalihydroxiden, wie LiOH, NaOH, KOH, Ca(OH)₂ oder Ba(OH)₂, bevorzugt NaOH oder KOH ein. Die Hydroxide werden dabei in Form einer 5 bis 20 %igen wäßrigen Lösung verwendet.

Die molaren Verhältnisse in denen Ester XI oder XII und Hydroxide eingesetzt werden, betragen z.B. 1:0,95 bis 1:1 für Alkalimetallhydroxide und 1:0,48 bis 1:0,55 für Erdalkalimetallhydroxide.

Eine weitere Möglichkeit, die als Ausgangsstoffe benötigten Dicarbonsäuremonoamide zu erhalten, besteht darin, daß man Dicarbonsäureanhydride der allgemeinen Formel VI bzw. VII in an sich bekannter Weise mit einem Amin VIII umsetzt und die entstehenden Stellungsisomeren chromatographisch oder durch fraktionierte Kristallisation trennt.

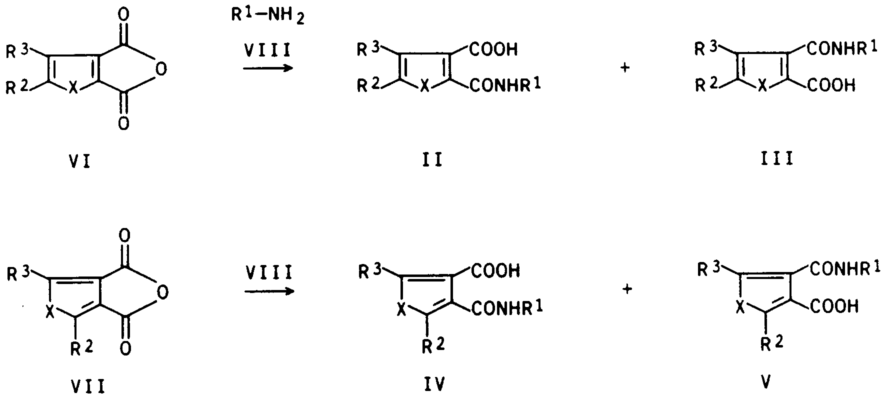

Die Umsetzung wird in der Regel bei Temperaturen von -10 bis 100°C, vorzugsweise 0 bis 30°C in einem inerten organischen Lösungsmittel durchgeführt.

Als Lösungsmittel eignen sich Ether wie Methyl-tert.-butylether, 1,2-Dimethoxyethan, Diethylenglykol-dimethylether, Tetrahydrofuran und Dioxan.

Die Konzentration der Edukte im Lösungsmittel beträgt im allgemeinen 0,1 bis 5 mol/l, bevorzugt 0,2 bis 2 mol/l.

Das molare Verhältnis VI bzw. VII zu VIII beträgt im allgemeinen 1 : 5 bis 1 : 1, vorzugsweise 1 : 2 bis 1 : 1.

Die benötigten Dicarbonsäureanhydride VI bzw. VII sind bekannt oder lassen sich in an sich bekannter Weise durch die Umsetzung der entsprechenden Dicarbonsäuren mit dem Anhydrid einer niederen Carbonsäure wie insbesondere Essigsäureanhydrid herstellen.

### Weg B:

Ein weiteres Verfahren zur Herstellung von Verbindungen der Formeln Ia und Ib besteht darin, daß man ein Dicarbonsäureanhydrid der Formel VI bzw. VII mit einem Amin der Formel VIII umsetzt. Die Reaktion wird zweckmäßigerweise so durchgeführt, daß man das Anhydrid VI bzw. VII in einem Lösungsmittel bei einer Temperatur von 0 - 150°C, vorzugsweise 20 - 100°C erhitzt. Als Lösungsmittel eignen sich z.B. niedere Alkansäuren wie Essigsäure, Propionsäure und Isobuttersäure und Ester dieser Säuren wie Essigsäureethylester, desweiteren aprotische Lösungsmittel wie Toluol und Xylol und/oder Dimethylformamid. Beim Arbeiten in einem aprotischen Lösungsmittel empfiehlt es sich, saure Katalysatoren wie z.B. aromatischen Sulfonsäuren zuzusetzen und das Reaktionswasser fortlaufend zu entfernen. Die molaren Verhältnisse, in denen die benötigten Ausgangsverbindungen miteinander umgesetzt werden, betragen im allgemeinen 0,9 bis 5, vorzugsweise 0,9 bis 1,5 für das Verhältnis von Anhydrid zu Amin.

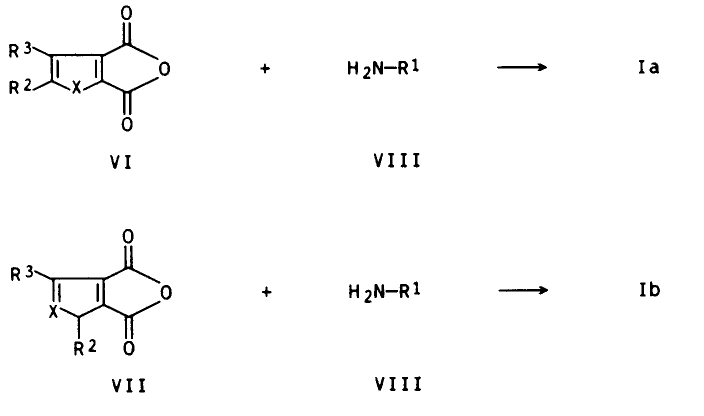

Im Hinblick auf die bestimmungsgemäße Verwendung der Verbindungen IA und IB kommen als Substituenten folgende Reste in Betracht:
X
   Sauerstoff oder Schwefel
R¹
   Wasserstoff, Hydroxyl;
   C₃-C₈-Cycloalkyl wie Cyclopropyl, Cyclobutyl, Cyclopentyl, Cyclohexyl, Cycloheptyl und Cyclooctyl, insbesondere Cyclopropyl, Cyclobutyl, Cyclopentyl und Cyclohexyl, welches ein bis drei der folgenden Reste tragen kann: Halogen wie Fluor, Chlor, Brom und Iod, insbesondere Fluor und Chlor; Alkyl wie Methyl, Ethyl, Propyl, 1-Methylethyl, Butyl, 1-Methylpropyl, 2-Methylpropyl und 1,1-Dimethylethyl, insbesondere Methyl und Ethyl; Halogenalkyl wie Fluormethyl, Difluormethyl, Trifluormethyl, Chlordifluormethyl, Dichlorfluormethyl, Trichlormethyl, 1-Fluorethyl, 2-Fluorethyl, 2,2-Difluorethyl, 2,2,2-Trifluorethyl, 2-Chlor-2,2-difluorethyl, 2,2-Dichlor-2-fluorethyl, 2,2,2-Trichlorethyl und Pentafluorethyl, insbesondere Trifluormethyl; Alkoxy wie Methoxy, Ethoxy, n-Propoxy, 2-Methylethoxy, n-Butoxy, 1-Methylpropoxy, 2-Methylpropoxy und 1,1-Dimethylethoxy, insbesondere Methoxy und Ethoxy; Halogenalkoxy, wie Difluormethoxy, Trifluormethoxy, Chlordifluormethoxy, Dichlorfluormethoxy, 1-Fluorethoxy, 2-Fluorethoxy, 2,2-Difluorethoxy, 1,1,2,2-Tetrafluorethoxy, 2,2,2-Trifluorethoxy, 2-Chlor-1,1,2-trifluorethoxy und Pentafluorethoxy, insbesondere Trifluormethoxy;
   C₁-C₆-Alkyl wie vorstehend genannt, sowie Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl, insbesondere Methyl, Ethyl, 1-Methylethyl und 1,1-Dimethylethyl, welches ein bis drei der folgenden Reste tragen kann: Hydroxy; Halogen wie vorstehend genannt, insbesondere Fluor und Chlor; Cyano; Cycloalkyl wie vorstehend genannt, insbesondere Cyclopropyl; Alkoxy wie vorstehend genannt, insbesondere Methoxy und Ethoxy; Halogenalkoxy wie vorstehend genannt, insbesondere Trifluormethoxy; Alkylthio, wie Methylthio, Ethylthio, n-Propylthio, 1-Methylethylthio, n-Butylthio, 1-Methylpropylthio, 2-Methylpropylthio und 1,1-Dimethylethylthio, insbesondere Methylthio und Ethylthio; Halogenalkylthio wie Difluormethylthio, Trifluormethylthio, Chlordifluormethylthio, 1-Fluorethylthio, 2-Fluorethylthio, 2,2-Difluorethylthio, 2,2,2-Trifluorethylthio, 2-Chlor-2,2-difluorethylthio, 2,2-Dichlor-2-fluorethylthio, 2,2,2-Trichlorethylthio und Pentafluorethylthio, insbesondere Trifluormethylthio und Pentafluorethylthio; Alkylamino wie Methylamino, Ethylamino, Propylamino, iso-Propylamino, insbesondere Methylamino; Dialkylamino wie Dimethylamino, Diethylamino, Dirpropylamino, Diisopropylamino, Methylethylamino, insbesondere Dimethylamino; Cycloalkylamino wie Cyclopropylamino, Cyclobutylamino, Cyclopentylamino und Cyclohexylamino, insbesondere Cyclopropylamino; und/oder einen Rest 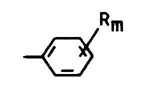 tragen kann, wobei
   - R: Cyano; Nitro; Halogen wie insbesondere Fluor und Chlor; C₁-C₄-Alkyl wie insbesondere Methyl, Ethyl und 1-Methylethyl; C₁-C₄-, insbesondere C₁-C₂-Halogenalkyl wie insbesondere Trifluormethyl; C₁-C₄-Alkoxy wie insbesondere Methoxy, Ethoxy und 1-Methylethoxy; C₁-C₄-Halogenalkoxy wie insbesondere Difluormethoxy und Trifluormethoxy; C₂-C₄-Alkinyloxy wie Propargyloxy; C₁-C₄-Alkylthio wie insbesondere Methylthio und Ethylthio; C₁-C₄-Halogenalkylthio wie insbesondere Difluormethylthio und Trifluormethylthio, C₁-C₄-Alkoxycarbonylalkoxy wie insbesondere Methoxy- oder Ethoxycarbonylmethoxy, C₁-C₄-Alkoxycarbonyl wie Methoxycarbonyl, Ethoxycarbonyl, Propyloxycarbonyl, 1-Methylethoxycarbonyl, Butyloxycarbonyl, 1-Methylpropyloxycarbonyl, 2-Methylpropyloxycarbonyl und 1,1-Dimethylethoxycarbonyl, insbesondere Methoxycarbonyl und Ethoxycarbonyl, 2-Alkoxycarbonylprop-1-enyl, C₁-C₄-Alkanoyl, wie Acetyl, C₁-C₄-Halogenalkanoyl wie Trifluor- und Trichloracetyl, sowie Formyl, geschütztes Formyl wie Dioxolanyl und/oder Phenyl bedeutet und
   - m: für 0, 1, 2 oder 3 steht, wobei die Reste R verschieden sein können, wenn m 2 oder 3 bedeutet;
   C₁-C₄-Alkoxy wie insbesondere Methoxy und Ethoxy;
   C₂-C₆-Cyanalkyl wie Cyanmethyl, Cyanbutyl, 2-Cyan-3-methylbut-2-yl und insbesondere 1,1-Dimethyl-cyanmethyl oder 1,1-Diethyl-cyanmethyl;
   C₃-C₆-Alkenyl wie 2-Propenyl, 2-Butenyl, 3-Butenyl, 1-Methyl-2-propenyl, 2-Methyl-2-propenyl, 2-Pentenyl, 3-Pentenyl, 4-Pentenyl, 3-Methyl-2-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-2-propenyl, 1-Ethyl-2-propenyl, 2-Hexenyl, 3-Hexenyl, 4-Hexenyl, 5-Hexenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-2-butenyl, 1,2-Dimethyl-3-butenyl, 1,3-Dimethyl-2-butenyl, 1,3-Dimethyl-3-butenyl, 2,2-Dimethyl-3-butenyl, 2,3-Dimethyl-2-butenyl, 2,3-Dimethyl-3-butenyl, 1-Ethyl-2-butenyl, 1-Ethyl-3-butenyl, 2-Ethyl-2-butenyl, 2-Ethyl-3-butenyl, 1,1,2-Trimethyl-2-propenyl, 1-Ethyl-1-methyl-2-propenyl und 1-Ethyl-2-methyl-2-propenyl; besonders bevorzugt 2-Propenyl;
   Alkinyl wie 2-Propinyl, 2-Butinyl, 3-Butinyl, 1-Methyl-2-propinyl, 2-Pentinyl, 3-Pentinyl, 4-Pentinyl, 1-Methyl-3-butinyl, 2-Methyl-3-butinyl, 1-Methyl-2-butinyl, 1,1-Dimethyl-2-propinyl, 1-Ethyl-2-propinyl, 2-Hexinyl, 3-Hexinyl, 4-Alkinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-4-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 1-Ethyl-2-butinyl, 1-Ethyl-3-butinyl, 2-Ethyl-3-butinyl und 1-Ethyl-1-methyl-2-propinyl; insbesondere 2-Propinyl;
   Phenyl und Naphthyl, wobei diese Gruppen ein bis drei der bei R im allgemeinen und im besonderen genannten Reste tragen können, wie z.B. 2-Fluorphenyl, 3-Fluorphenyl, 4-Fluorphenyl, 2-Chlorphenyl, 3-Chlorphenyl, 4-Chlorphenyl, 2-Methoxyphenyl, 3-Methoxyphenyl, 4-Methoxyphenyl, 2-Trifluormethylphenyl, 3-Trifluormethylphenyl, 4-Trifluormethylphenyl;
   einen 5- bis 6-gliedrigen Heterocyclus enthaltend ein oder zwei Heteroatome, ausgewählt aus der Gruppe Stickstoff, Sauerstoff und Schwefel, wie 2-Tetrahydrofuranyl, 3-Tetrahydrofuranyl, 2-Tetrahyrothienyl, 3-Tetrahydrothienyl, 2-Tetrahydropyranyl, 3-Tetrahydropyranyl, 4-Tetrahydropyranyl, 2-Furanyl, 3-Furanyl, 2-Thienyl, 3-Thienyl, 3-Isoxazolyl, 4-Isoxazolyl, 5-Isoxazolyl, 3-Isothiazolyl, 4-Isothiazolyl, 5-Isothiazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 5-Imidazolyl, 2-Pyrrolyl, 4-Pyrrolyl, 3-Pyrazolyl, 4-Pyrazolyl, 5-Pyrazolyl, 2-Pyridyl, 3-Pyridyl, 4-Pyridyl und 2-(4,6-Dimethyl-pyrimidinyl), wobei dieser Ring ein oder zwei der folgenden Reste tragen kann: Halogen, Alkyl, Halogenalkyl, Alkoxy, Halogenalkoxy und/oder Alkylthio wie im allgemeinen und im besonderen bei R¹ genannt,
   Di-C₁-C₄-alkylamino, insbesondere Dimethyl- und Diethylamino;
R2, R³
   Nitro; Cyano;
   Halogen wie insbesondere Fluor, Chlor und Brom;
   Amino, welches ein oder zwei C₁-C₄-Alkylgruppen wie bei R¹ genannt, insbesondere Methyl und Ethyl und/oder eine C₁-C₄-Alkylcarbonylgruppe wie Methylcarbonyl, Ethylcarbonyl, Propylcarbonyl, 1-Methylethylcarbonyl, Butylcarbonyl, 1-Methylpropylcarbonyl, 2-Methylpropylcarbonyl und 1,1-Dimethylethylcarbonyl, insbesondere Methylcarbonyl und Ethylcarbonyl tragen kann;
   C₁-C₄-Alkoxy oder Alkylthio wie insbesondere Methoxy, Ethoxy, Methylthio und Ethylthio, wobei diese Gruppen ein bis neun Halogenatome wie insbesondere Fluor und Chlor tragen können, z.B. Difluormethoxy, Trifluormethoxy, Chlordifluormethoxy, Dichlorfluormethoxy, 1-Fluorethoxy, 2-Fluorethoxy, 2,2-Difluorethoxy, 1,1,2,2,-Tetrafluorethoxy, 2,2,2-Trifluorethoxy, 2-Chlor-1,1,2-trifluorethoxy und Pentafluorethoxy, insbesondere Trifluormethoxy und Trifluormethylthio;
   C₂-C₆-Alkenyl wie Ethenyl, 1-Propenyl, 1-Methylethenyl, 1-Butenyl, 1-Methyl-1-propenyl, 2-Methyl-1-propenyl, 1-Pentenyl, 1-Methyl-1-butenyl, 2-Methyl-1-butenyl, 1,1-Dimethyl-1-propenyl, 1-Ethyl-1-propenyl, 1-Hexenyl, 1-Methyl-1-pentenyl, 2-Methyl-1-pentenyl, 3-Methyl-1-pentenyl, 4-Methyl-1-pentenyl, 1,2-Dimethyl-1-butenyl, 1,3-Dimethyl-1-butenyl, 2,3-Dimethyl-1-butenyl, 3,3-Dimethyl-1-butenyl, 1-Ethyl-1-butenyl, 2-Ethyl-1-butenyl, 1-Ethyl-2-methyl-1-propenyl, insbesondere 2-Propenyl;
   C₂-C₆-Alkinyl wie Ethinyl, 1-Propinyl, 1-Butinyl, 1-Pentinyl, 1-Methyl-3-butinyl, 1-Hexinyl, 3-Methyl-1-pentinyl, 4-Methyl-1-pentinyl, 3,3-Dimethyl-1-butinyl, insbesondere 2-Propinyl;
   C₁-C₄-Alkyl- oder Halogenalkylsulfonyl wie insbesondere Methylsulfonyl, Trifluormethylsulfonyl und Trichlormethylsulfonyl;
   Phenyl, Phenoxy oder Phenylthio, wobei diese Gruppen ein bis drei der im allgemeinen und im besonderen bei R genannten Reste tragen können, wie 2-Fluorphenyl, 3-Fluorphenyl, 4-Fluorphenyl, 2-Chlorphenyl, 3-Chlorphenyl, 4-Chlorphenyl, 2-Methoxyphenyl, 3-Methoxyphenyl, 4-Methoxyphenyl, 2-Trifluormethylphenyl, 3-Trifluormethylphenyl, 4-Trifluormethylphenyl,
   oder eine der im allgemeinen und im besonderen bei R¹ genannten Gruppen;

Beispiele für herbizid wirksame Verbindungen der Formeln Ia und Ib sind nachstehend im einzelnen aufgeführt: 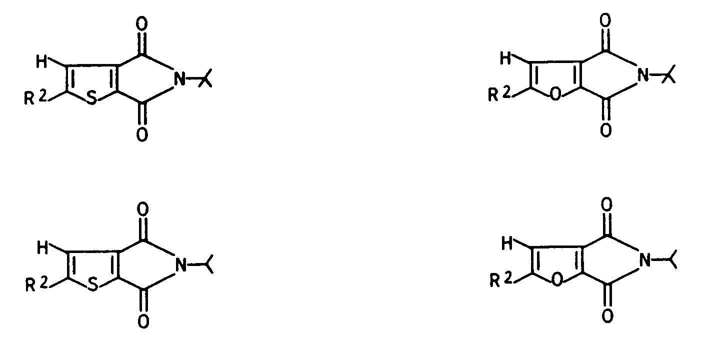 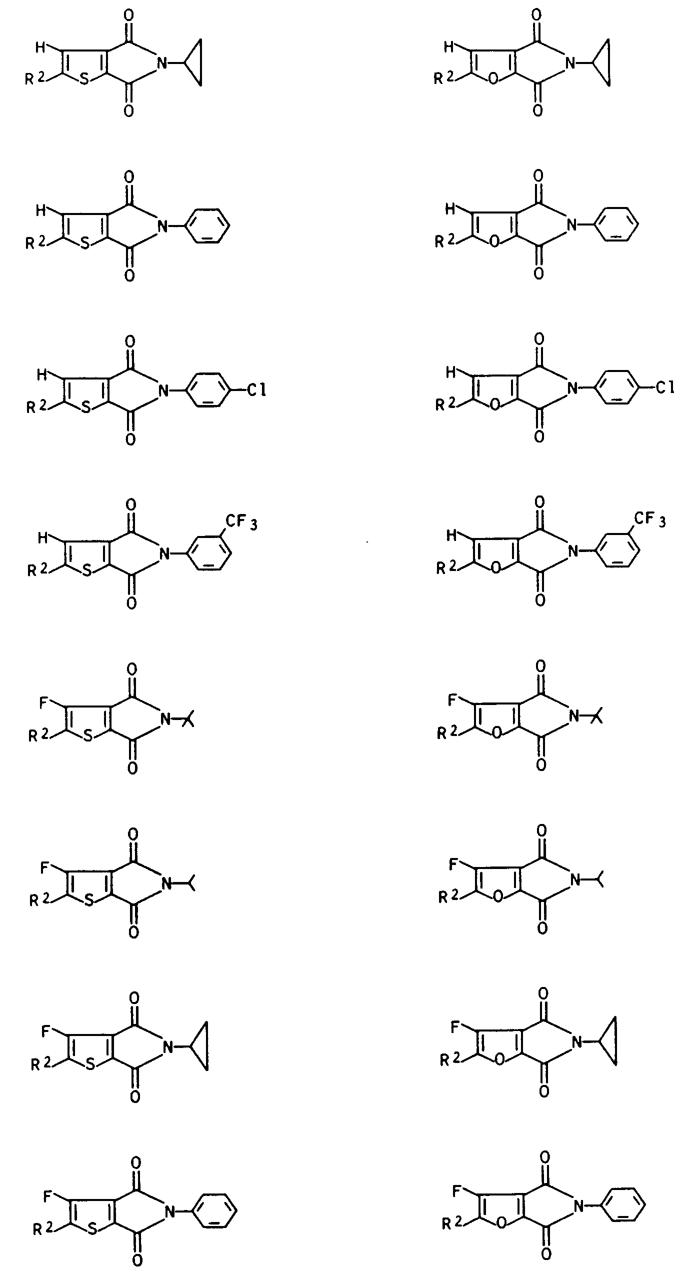 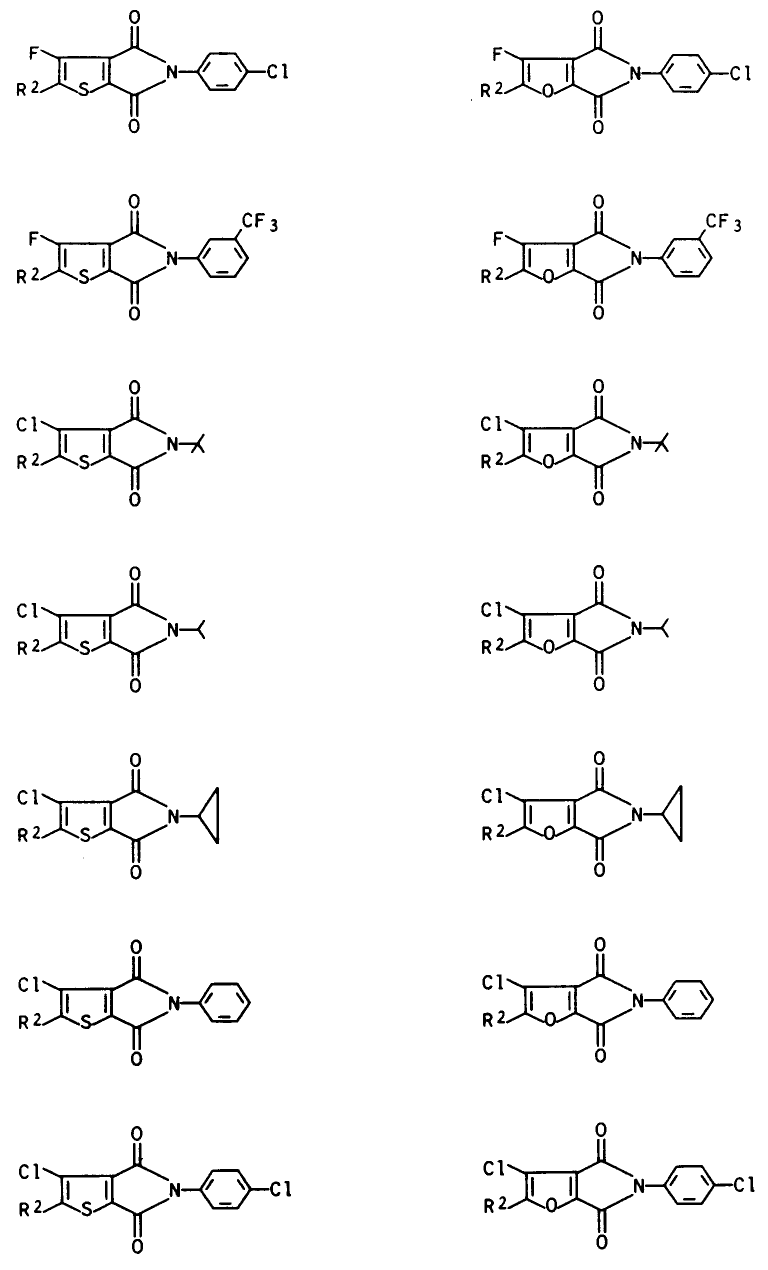 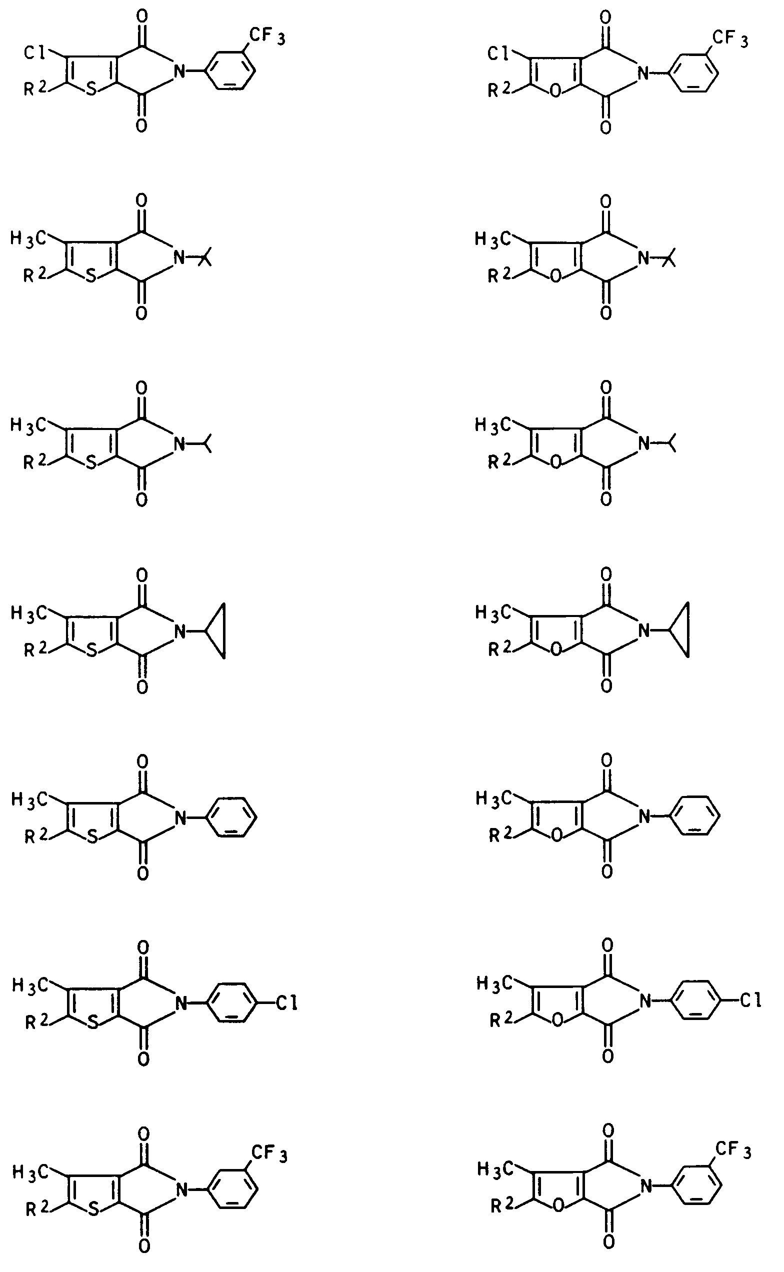 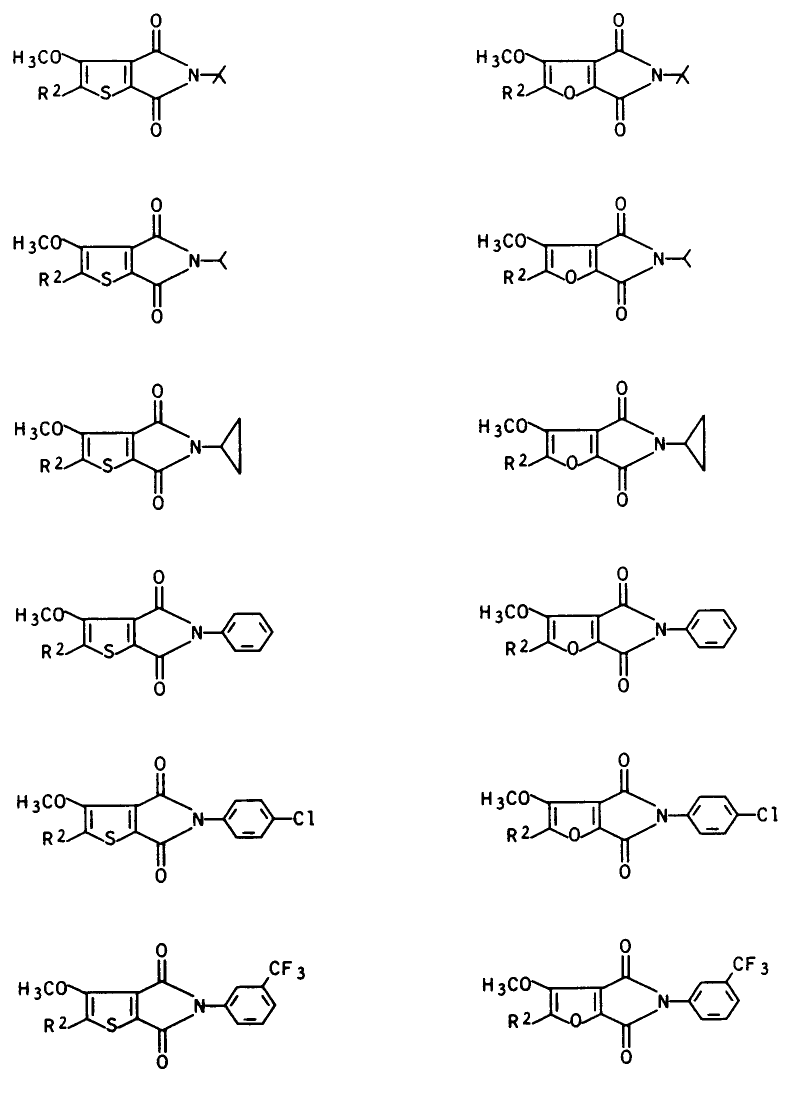
wobei R² jeweils die folgende Bedeutung hat:
Wasserstoff, Fluor, Chlor, Brom, Iod, Cyano, Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 1,1-Dimethylpropyl, 1,2-Dimethylpropyl, 2,2-Dimethylpropyl, cyclo-Propyl, cyclo-Butyl, cyclo-Pentyl, cyclo-Hexyl, cyclo-Heptyl, cyclo-Octyl, 1-Methyl-cyclo-Propyl, cyclo-Propyl-methyl, 1-(cyclo-Propyl)-ethyl, Chlormethyl, Dichlormethyl, Trichlormethyl, Chlordifluormethyl, Trifluormethyl, Pentafluorethyl, Difluormethyl, 1-Chlorethyl, 2-Chlorethyl, 1-Methyl-1-chlorethyl, 1-Methyl-2-chlorethyl, Methoxymethyl, 1-Methylmethoxymethyl, 1-Methyl-2-methoxyethyl, 1-Methylethoxymethyl, Ethoxymethyl, Ethenyl, 1-Propenyl, 2-Propenyl, 1-Methylethenyl, 1-Ethylethenyl, 2-Phenylethenyl, 1-Butenyl, 2-Butenyl, 3-Butenyl, 1-Methyl-1-propenyl, 1-Methyl-2-propenyl, 2-Methyl-2-propenyl, 2-Propinyl, Methoxy, Ethoxy, Propoxy, 1-Methylethoxy, Butoxy, 1-Methylpropoxy, 1,1-Dimethylethoxy, Methylthio, Ethylthio, Chlordifluormethoxy, Trifluor-methoxy, Trichlormethylthio, Phenyl, 2-Fluorphenyl, 3-Fluorphenyl, 4-Fluorphenyl, 2-Chlorphenyl, 3-Chlorphenyl, 4-Chlorphenyl, 2-Methylphenyl, 3-Methylphenyl, 4-Methylphenyl, 2-Trifluormethylphenyl, 3-Trifluormethylphenyl, 4-Trifluormethylphenyl, 2-Methoxyphenyl, 3-Methoxyphenyl, 4-Methoxyphenyl, 2,4-Dichlorphenyl, 2,4,6-Trimethylphenyl, Phenoxy, Phenylthio, 2-Chlorphenoxy, 3-Chlorphenoxy, 4-Chlorphenoxy, 2,4-Dichlorphenoxy, Benzyl, 2-Chlorbenzyl, 3-Chlorbenzyl, 4-Chlorbenzyl, 2-Fluorbenzyl, 3-Fluorbenzyl, 4-Fluorbenzyl, 2-Thienyl, 3-Thienyl, 2-Pyridyl, 3-Pyridyl, 4-Pyridyl. 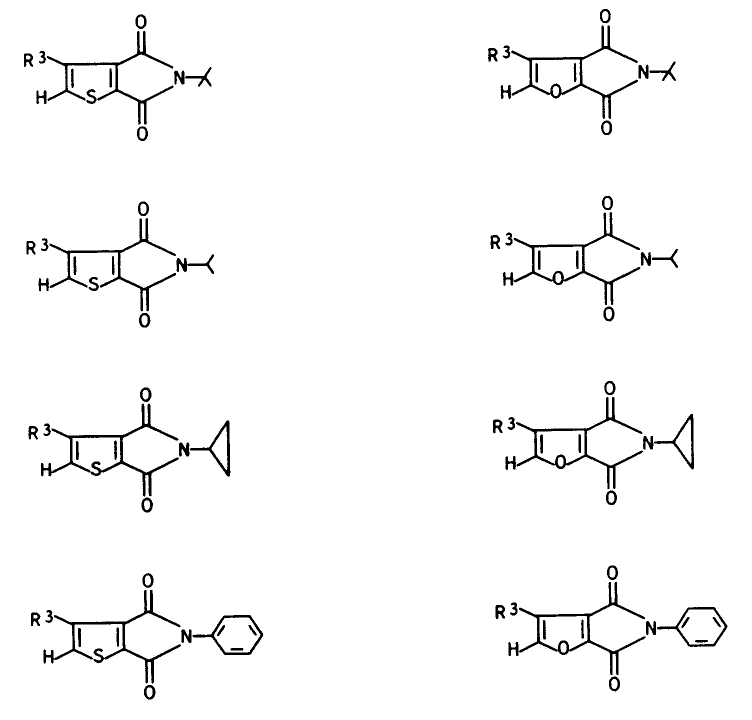 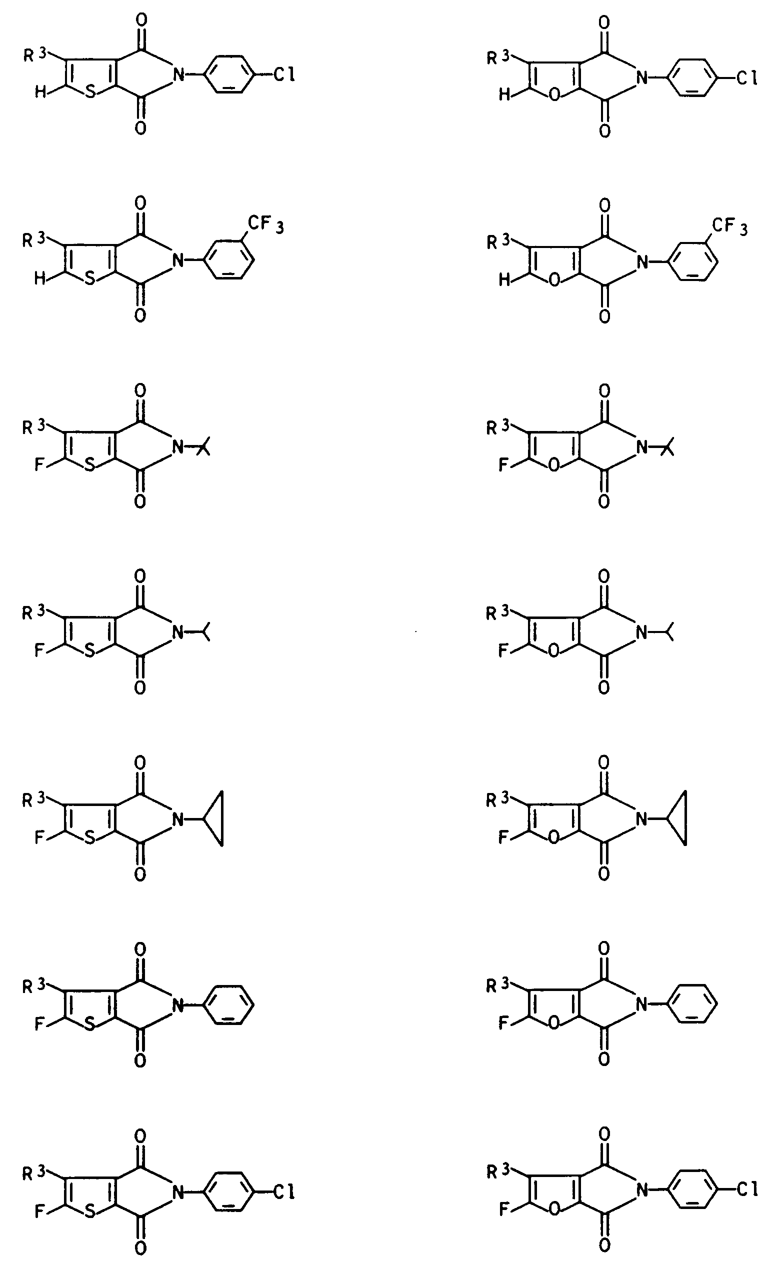 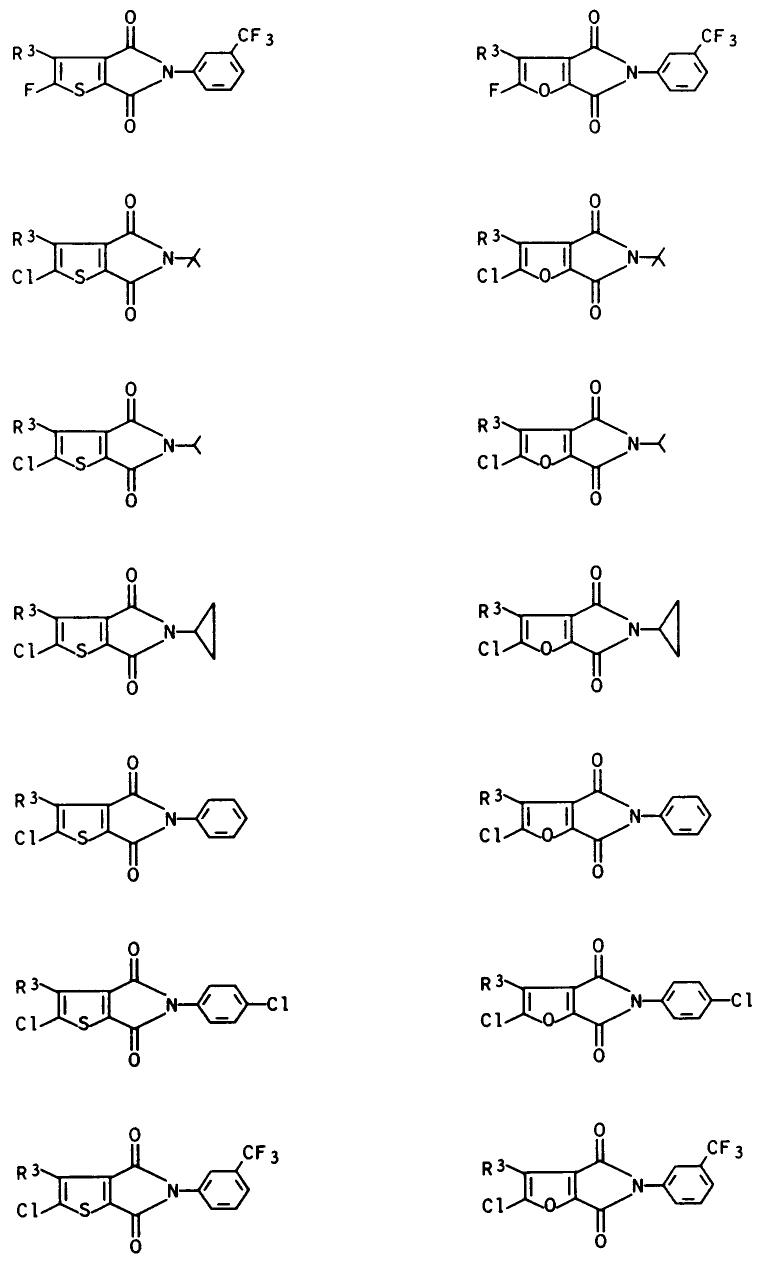 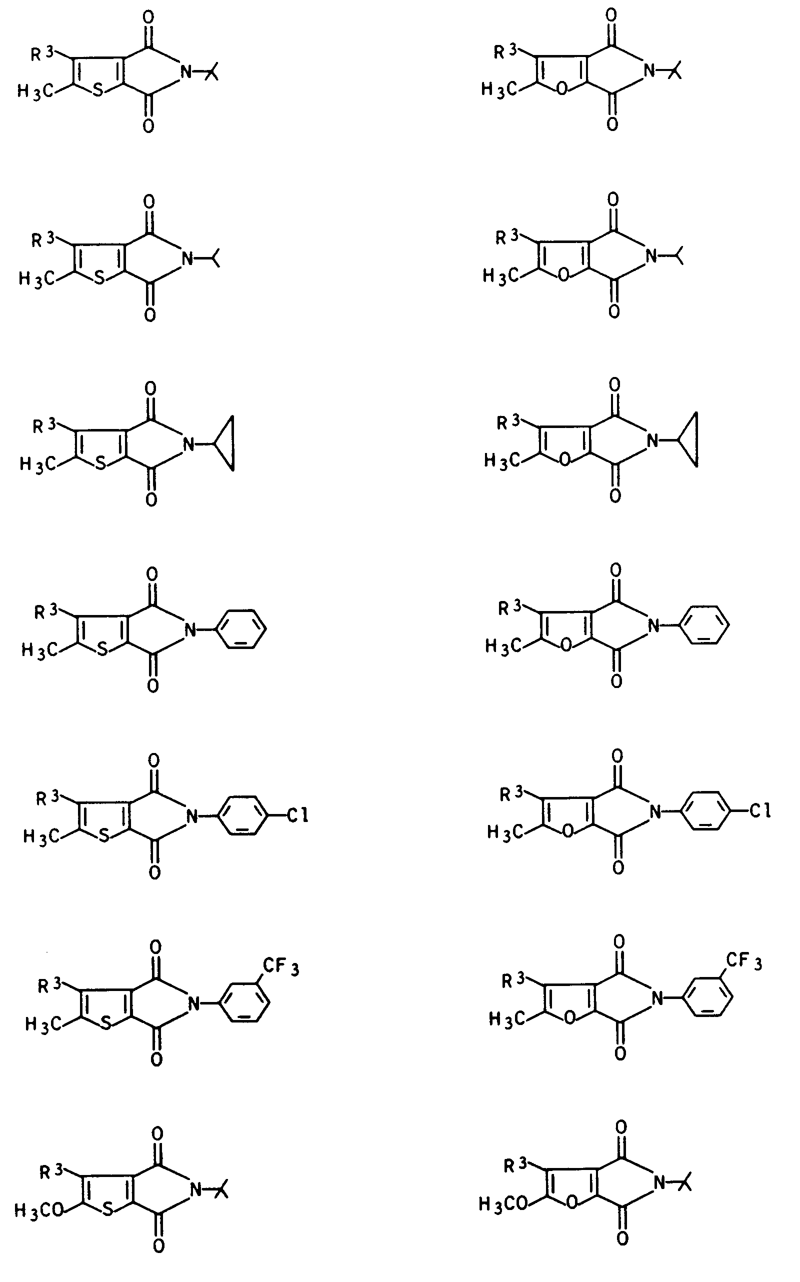 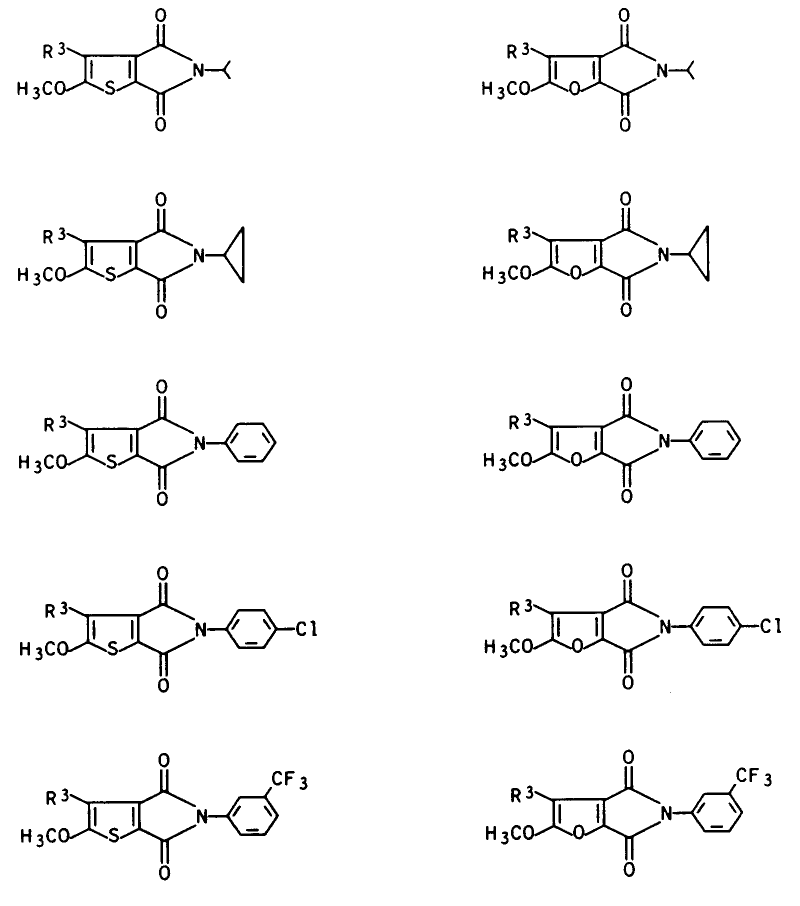
wobei R³ jeweils die folgende Bedeutung hat:
Wasserstoff, Fluor, Chlor, Brom, Iod, Cyano, Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 1,1-Dimethylpropyl, 1,2-Dimethylpropyl, 2,2-Dimethylpropyl, cyclo-Propyl, cyclo-Butyl, cyclo-Pentyl, cyclo-Hexyl, cyclo-Heptyl, cyclo-Octyl, 1-Methyl-cyclo-Propyl, cyclo-Propyl-methyl, 1-(cyclo-Propyl)-ethyl, Chlormethyl, Dichlormethyl, Trichlormethyl, Chlordifluormethyl, Trifluormethyl, Pentafluorethyl, Difluormethyl, 1-Chlorethyl, 2-Chlorethyl, 1-Methyl-1-chlorethyl, 1-Methyl-2-chlorethyl, Methoxymethyl, 1-Methylmethoxymethyl, 1-Methyl-2-methoxyethyl, 1-Methylethoxymethyl, Ethoxymethyl, Ethenyl, 1-Propenyl, 2-Propenyl, 1-Methylethenyl, 1-Ethylethenyl, 2-Phenylethenyl, 1-Butenyl, 2-Butenyl, 3-Butenyl, 1-Methyl-1-propenyl, 1-Methyl-2-propenyl, 2-Methyl-2-propenyl, 2-Propinyl, Methoxy, Ethoxy, Propoxy, 1-Methylethoxy, Butoxy, 1-Methylpropoxy, 1,1-Dimethylethoxy, Methylthio, Ethylthio, Chlordifluormethoxy, Trifluor-methoxy, Trichlormethylthio, Phenyl, 2-Fluorphenyl, 3-Fluorphenyl, 4-Fluorphenyl, 2-Chlorphenyl, 3-Chlorphenyl, 4-Chlorphenyl, 2-Methylphenyl, 3-Methylphenyl, 4-Methylphenyl, 2-Trifluormethylphenyl, 3-Trifluormethylphenyl, 4-Trifluormethylphenyl, 2-Methoxyphenyl, 3-Methoxyphenyl, 4-Methoxyphenyl, 2,4-Dichlorphenyl, 2,4,6-Trimethylphenyl, Phenoxy, Phenylthio, 2-Chlorphenoxy, 3-Chlorphenoxy, 4-Chlorphenoxy, 2,4-Dichlorphenoxy, Benzyl, 2-Chlorbenzyl, 3-Chlorbenzyl, 4-Chlorbenzyl, 2-Fluorbenzyl, 3-Fluorbenzyl, 4-Fluorbenzyl, 2-Thienyl, 3-Thienyl, 2-Pyridyl, 3-Pyridyl, 4-Pyridyl. 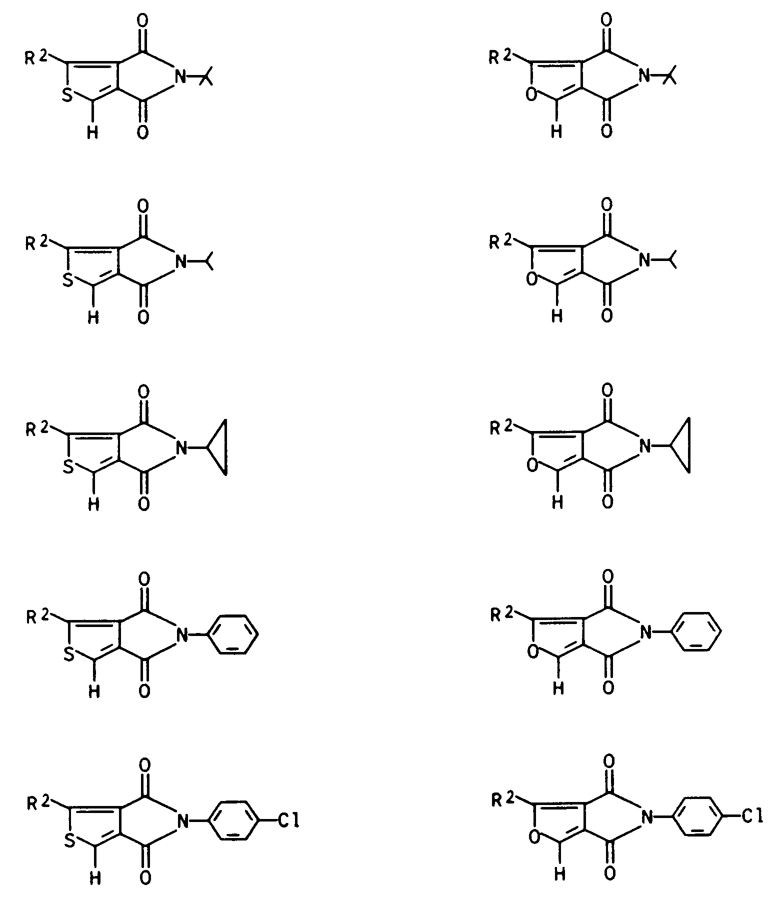 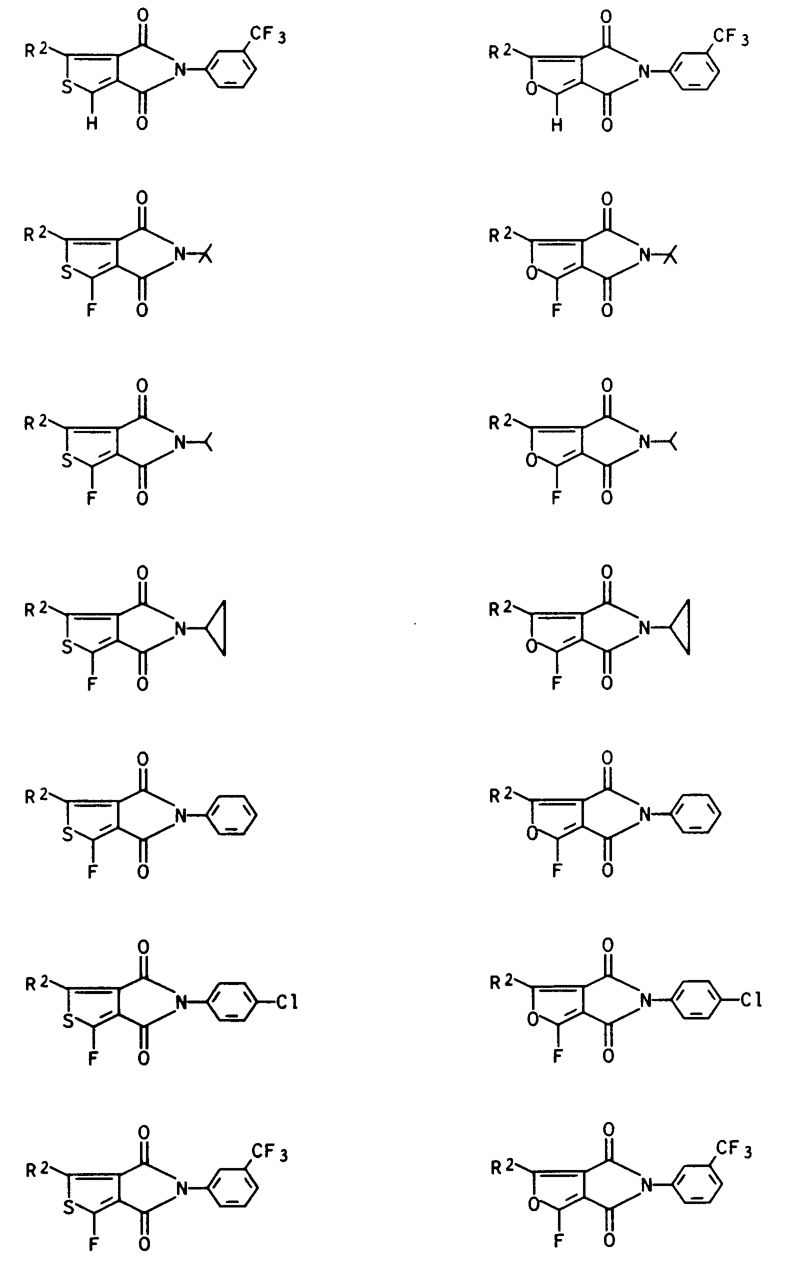 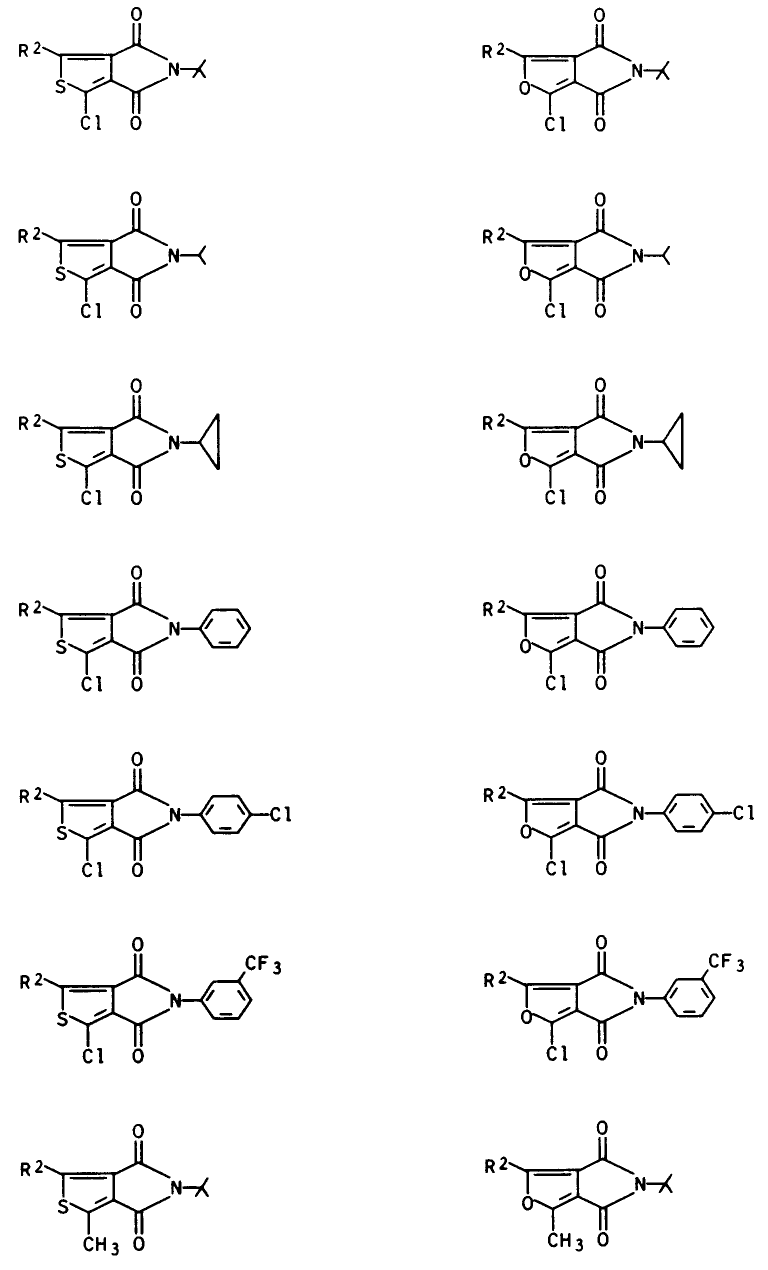 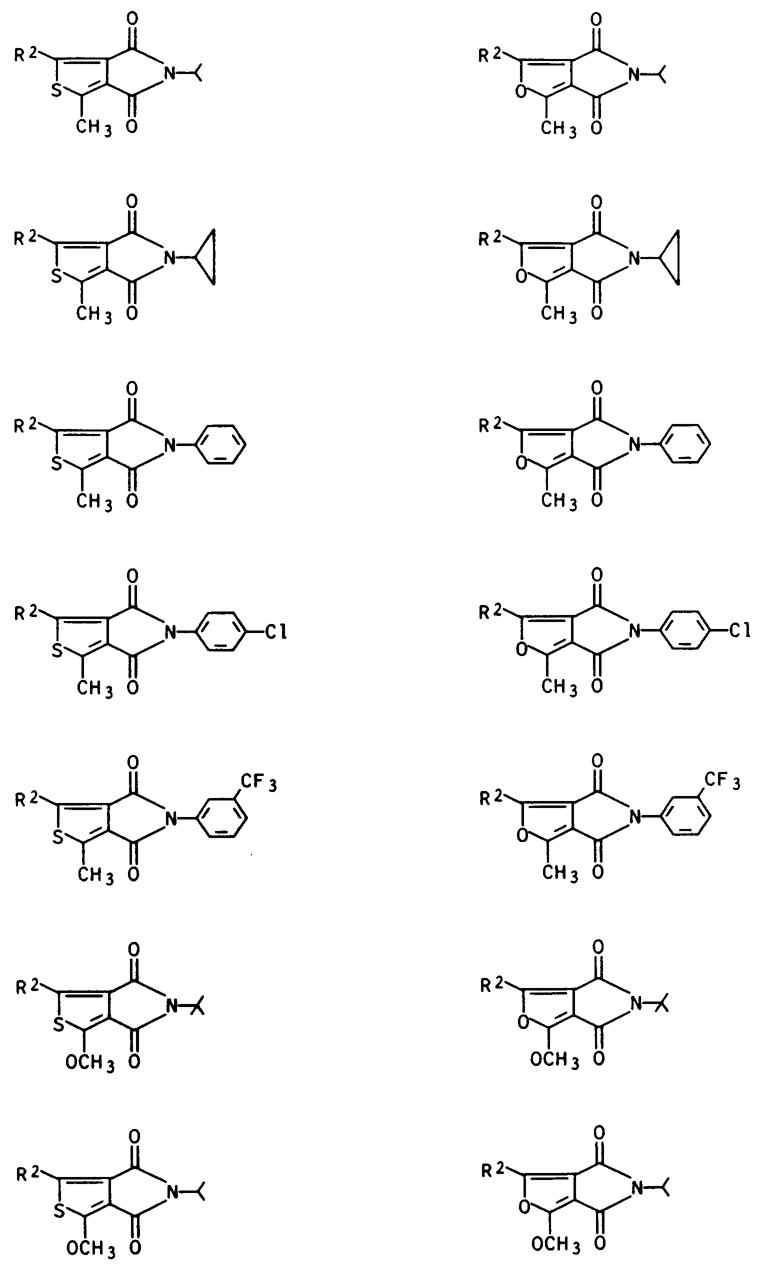 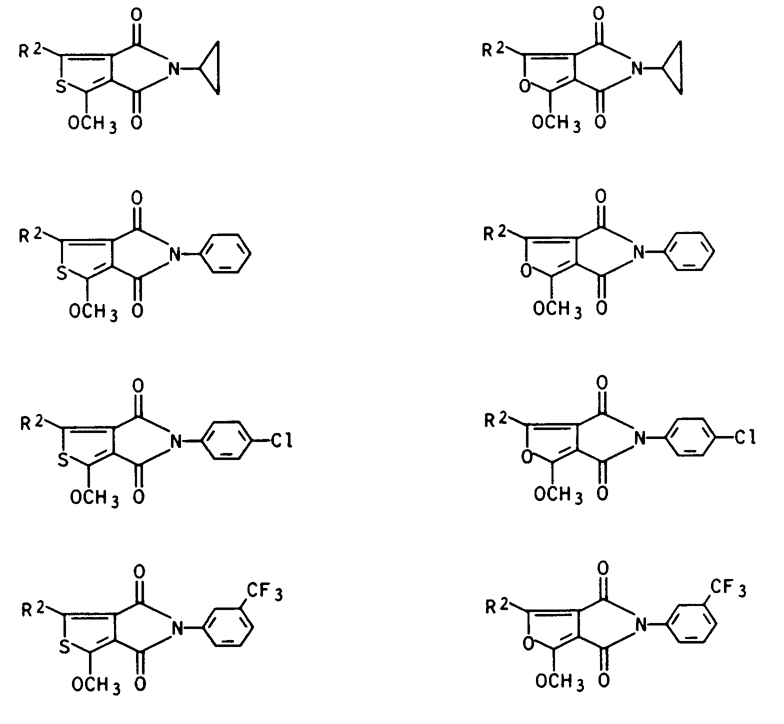
wobei R² jeweils die folgende Bedeutung hat:
Wasserstoff, Fluor, Chlor, Brom, Iod, Cyano, Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 1,1-Dimethylpropyl, 1,2-Dimethylpropyl, 2,2-Dimethylpropyl, cyclo-Propyl, cyclo-Butyl, cyclo-Pentyl, cyclo-Hexyl, cyclo-Heptyl, cyclo-Octyl, 1-Methyl-cyclo-Propyl, cyclo-Propyl-methyl, 1-(cyclo-Propyl)-ethyl, Chlormethyl, Dichlormethyl, Trichlormethyl, Chlordifluormethyl, Trifluormethyl, Pentafluorethyl, Difluormethyl, 1-Chlorethyl, 2-Chlorethyl, 1-Methyl-1-chlorethyl, 1-Methyl-2-chlorethyl, Methoxymethyl, 1-Methylmethoxymethyl, 1-Methyl-2-methoxyethyl, 1-Methylethoxymethyl, Ethoxymethyl, Ethenyl, 1-Propenyl, 2-Propenyl, 1-Methylethenyl, 1-Ethylethenyl, 2-Phenylethenyl, 1-Butenyl, 2-Butenyl, 3-Butenyl, 1-Methyl-1-propenyl, 1-Methyl-2-propenyl, 2-Methyl-2-propenyl, 2-Propinyl, Methoxy, Ethoxy, Propoxy, 1-Methylethoxy, Butoxy, 1-Methylpropoxy, 1,1-Dimethylethoxy, Methylthio, Ethylthio, Chlordifluormethoxy, Trifluor-methoxy, Trichlormethylthio, Phenyl, 2-Fluorphenyl, 3-Fluorphenyl, 4-Fluorphenyl, 2-Chlorphenyl, 3-Chlorphenyl, 4-Chlorphenyl, 2-Methylphenyl, 3-Methylphenyl, 4-Methylphenyl, 2-Trifluormethylphenyl, 3-Trifluormethylphenyl, 4-Trifluormethylphenyl, 2-Methoxyphenyl, 3-Methoxyphenyl, 4-Methoxyphenyl, 2,4-Dichlorphenyl, 2,4,6-Trimethylphenyl, Phenoxy, Phenylthio, 2-Chlorphenoxy, 3-Chlorphenoxy, 4-Chlorphenoxy, 2,4-Dichlorphenoxy, Benzyl, 2-Chlorbenzyl, 3-Chlorbenzyl, 4-Chlorbenzyl, 2-Fluorbenzyl, 3-Fluorbenzyl, 4-Fluorbenzyl, 2-Thienyl, 3-Thienyl, 2-Pyridyl, 3-Pyridyl, 4-Pyridyl. 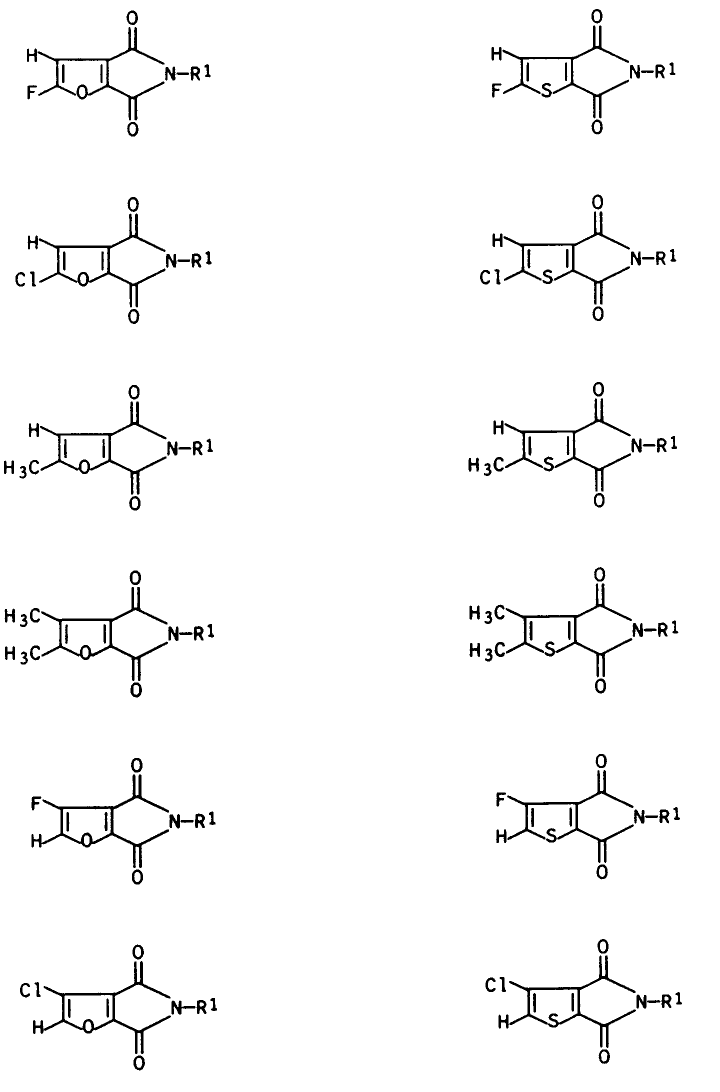 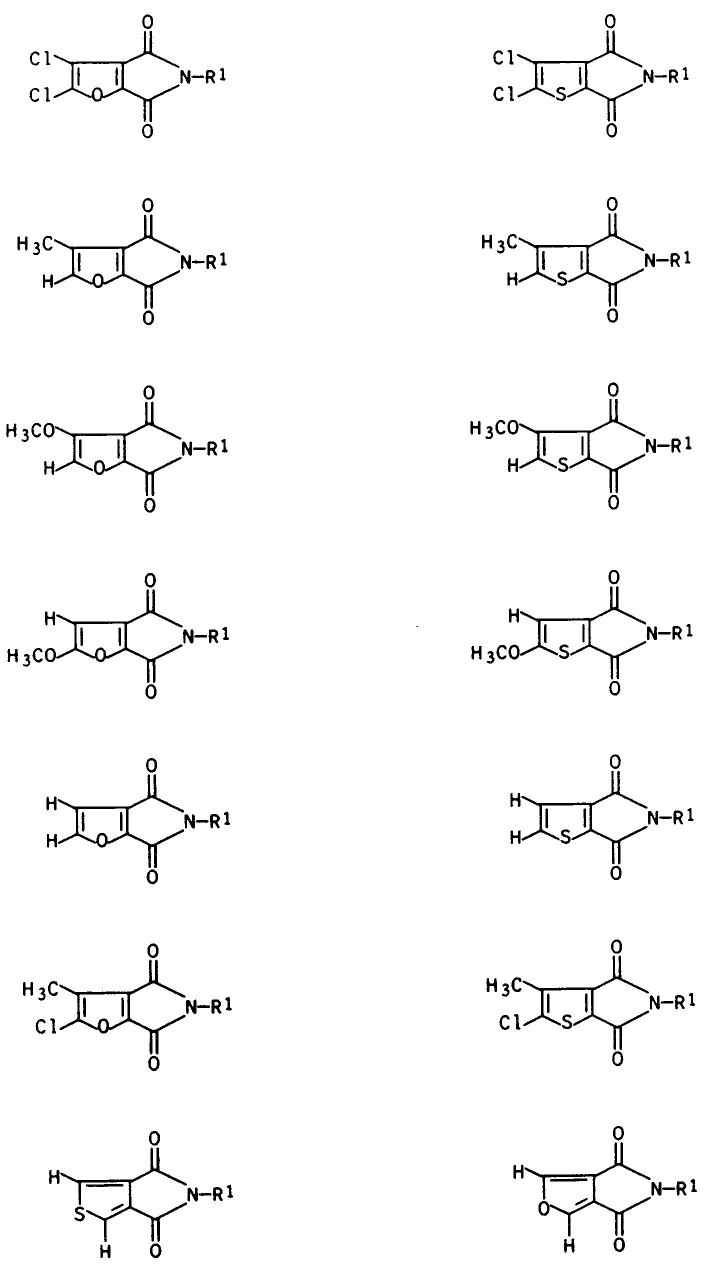 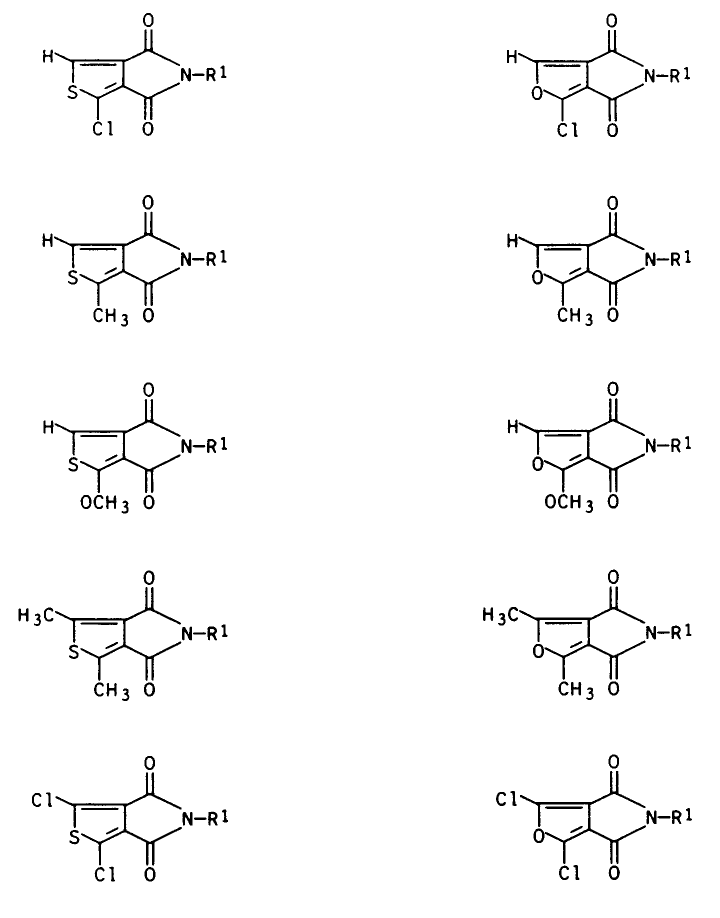
wobei R¹ jeweils die folgende Bedeutung hat:
Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, cyclo-Propyl, cyclo-Butyl, cyclo-Pentyl, cyclo-Hexyl, cyclo-Heptyl, cyclo-Octyl, 1-Methyl-cyclo-Propyl, cyclo-Propyl-methyl, 1-(cyclo-Propyl)-ethyl, 1-Methylcyclohexyl, 1-Ethylcyclohexyl, Cyclohexylmethyl, 2-Propenyl, 1-Methyl-2-propenyl, 1,1-Dimethyl-2-propenyl, 2-Propinyl, 1-Methyl-2-propinyl, 1,1-Dimethylpropinyl, Phenylmethyl, 1-Methylphenylmethyl, 1,1-Dimethylphenylmethyl, 2-Phenylethyl, 2-Methylthioethyl, 1-Methyl-2-methylthioethyl, 1,1-Dimethyl-2-methylthioethyl, 3-Methylthiopropyl, 2-Fluorethyl, 2-Fluor-1-methylethyl, 1,1-Dimethyl-2-fluorethyl, 2-Chlorethyl, 2-Chlor-1-methylethyl, 2-Chlor-1,1-Dimethylethyl, 2-Methoxyethyl, 2-Methoxy-1-methylethyl, 1,1-Dimethyl-2-methoxyethyl, 3-Methoxypropyl, 2-Cyanoethyl, 2-Cyano-1-methylethyl, 2-Cyano-1,1-dimethylethyl, Dimethylamino, Diethylamino, Morpholino, Piperidino, Phenyl, 2-Methylphenyl, 3-Methylphenyl, 4-Methylphenyl, 2-Ethylphenyl, 3-Ethylphenyl, 4-Ethylphenyl, 2,3-Dimethylphenyl, 2,4-Dimethylphenyl, 2,5-Dimethylphenyl, 2,6-Dimethylphenyl, 3,4-Dimethylphenyl, 3,5-Dimethylphenyl, 2,3,4-Trimethylphenyl, 2,3,5-Trimethylphenyl, 2,4,5-Trimethylphenyl, 2,4,6-Trimethylphenyl, 3,4,5-Trimethylphenyl, 2-Trifluormethylphenyl, 3-Trifluormethylphenyl, 4-Trifluormethylphenyl, 2-Fluorphenyl, 3-Fluorphenyl, 4-Fluorphenyl, 2-Chlorphenyl, 3-Chlorphenyl, 4-Chlorphenyl, 2,3-Difluorphenyl, 2,4-Difluorphenyl, 2,5-Difluorphenyl, 2,6-Difluorphenyl, 2,3-Dichlorphenyl, 2,4-Dichlorphenyl, 2,5-Dichlorphenyl, 2,6-Dichlorphenyl, 2,3,4-Trichlorphenyl, 2,3,5-Trichlorphenyl, 2,4,6-Trichlorphenyl, 3,4,5-Trichlorphenyl, 2-Cyanophenyl, 3-Cyanophenyl, 4-Cyanophenyl, 2-Methoxyphenyl, 3-Methoxyphenyl, 4-Methoxyphenyl, 2,3-Dimethoxyphenyl, 2,4-Dimethoxyphenyl, 2,5-Dimethoxyphenyl, 2,6-Dimethoxyphenyl, 3,4-Dimethoxyphenyl, 3,5-Dimethoxyphenyl, 3,4,5-Dimethoxyphenyl, 2-Trifluormethoxyphenyl, 3-Trifluormethoxyphenyl, 4-Trifluormethoxyphenyl, 2-Nitrophenyl, 3-Nitrophenyl, 4-Nitrophenyl, 2,3-Dinitrophenyl, 2,4-Dinitrophenyl, 2,5-Dinitrophenyl, 2,6-Dinitrophenyl, 3,4-Dinitrophenyl, 3,5-Dinitrophenyl, 1-Naphthyl, 2-Naphthyl, 3-Tetrahydrofuryl, 4-Tetrahydropyranyl, 2-Thiazolyl.

Die vorstehend genannten Restedefinitionen für R¹ und R² können darüberhinaus in den Furan- bzw. Thiophen-2,3- oder 3,4-dicarbonsäureimiden zu anderen als den aufgeführten Kombinationen miteinander kombiniert werden.

Als Salze der Verbindungen der Formeln Ia und Ib kommen landwirtschaftlich brauchbare Salze, beispielsweise Alkalimetallsalze, wie das Kalium- oder Natriumsalz, Erdalkalimetallsalze, wie das Calcium-, Magnesium- oder Bariumsalz, Mangan-, Kupfer-, Zink- oder Eisensalze sowie Ammonium, Phosphonium-, Sulfonium- oder Sulfoxoniumsalze, beispielsweise Ammoniumsalze, Tetraalkylammoniumsalze, Benzyltrialkylammoniumsalze, Trialkylsulfoniumsalze oder Trialkylsulfoxoniumsalze in Betracht.

Die erfindungsgemäßen herbiziden Verbindungen bzw. die sie enthaltenden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Die Verbindungen eignen sich allgemein zur Herstellung von direkt versprühbaren Lösungen, Emulsionen, Pasten oder Öldispersionen. Als inerte Zusatzstoffe kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Chlorbenzol, Isophoron oder stark polare Lösungsmittel wie N,N-Dimethylformamid, Dimethylsulfoxid, N-Methylpyrrolidon oder Wasser in Betracht.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylenalkylether, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Die Formulierungen enthalten zwischen 0,1 und 95 Gew.%, vorzugsweise zwischen 0,5 und 90 Gew.%, Wirkstoff. Die Wirkstoffe werden dabei in einer Reinheit von 90 % bis 100 %, vorzugsweise 95 % bis 100 % (nach NMR-Spektrum) eingesetzt.

Die erfindungsgemäßen Verbindungen I können beispielsweise wie folgt formuliert werden:
I. Man vermischt 90 Gewichtsteile der Verbindung Nr. 1.005 mit 10 Gewichtsteilen N-Methyl-α-pyrrolidon und erhält eine Lösung, die zur Anwendung in Form kleinster Tropfen geeignet ist.
II. 20 Gewichtsteile der Verbindung Nr. 2.007 werden in einer Mischung gelöst, die aus 80 Gewichtsteilen Xylol, 10 Gewichtsteilen des Anlagerungsproduktes von 8 bis 10 Mol Ethylenoxid an 1 Mol ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.% des Wirkstoffs enthält.
III. 20 Gewichtsteile der Verbindung Nr. 2.007 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.
IV. 20 Gewichtsteile des Wirkstoffs Nr. 1.005 werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.
V. 20 Gewichtsteile des Wirkstoffs Nr. 2.007 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gew.% des Wirkstoffs enthält.
VI. 3 Gewichtsteile des Wirkstoffs Nr. 1.005 werden mit 97 Gewichtsteilen feinteiligem Kaolin vermischt. Man erhält auf diese Weise ein Stäubemittel, das 3 Gew.% des Wirkstoffs enthält.
VII. 30 Gewichtsteile des Wirkstoffs Nr. 2.007 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.
VIII. 20 Gewichtsteile des Wirkstoffs Nr. 1.005 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.

Die Applikation der herbiziden Mittel bzw. der Wirkstoffe kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff bei Anwendung als Herbizide betragen je nach Bekämpfungsziel, Jahreszeit, Zielpflanzen und Wachstumsstadium 0,001 bis 3, vorzugsweise 0,01 bis 2 kg/ha aktive Substanz (a.S.).

In Anbetracht des erfaßbaren Wirkungsspektrums zur Unkrautbekämpfung, der Verträglichkeit für Kulturpflanzen oder der erwünschten Beeinflussung des Wachstums derselben sowie angesichts der Vielfalt der Applikationsmethoden können die erfindungsgemäßen Verbindungen in einer großen Zahl von Kulturpflanzen eingesetzt werden. In Betracht kommen beispielsweise folgende Kulturen:

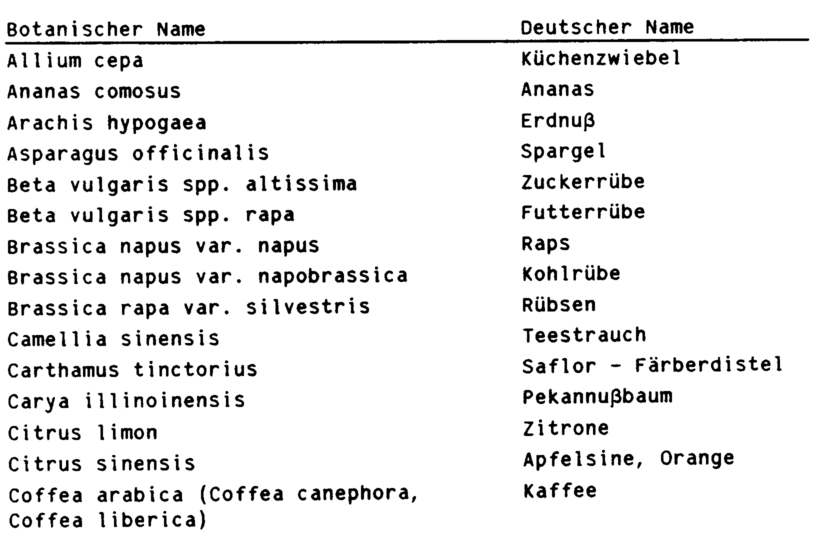

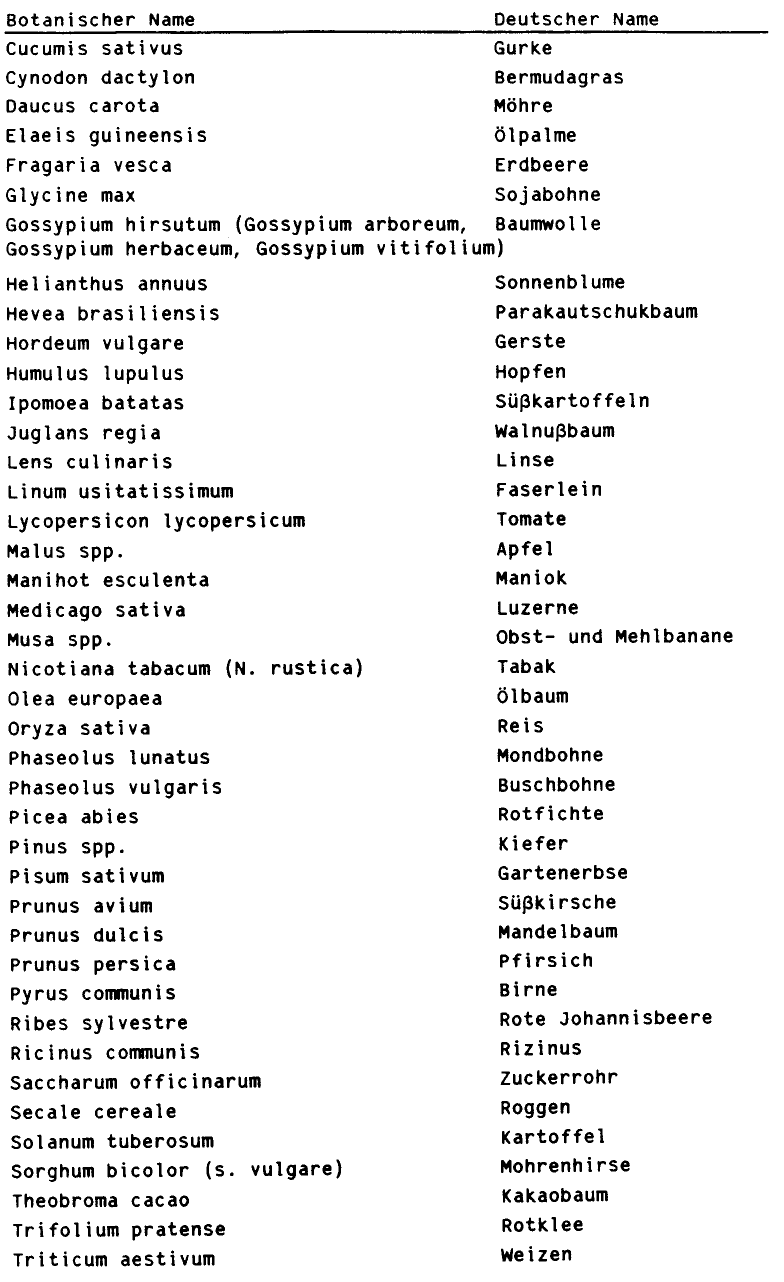

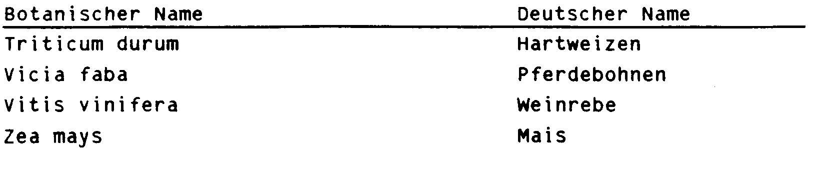

Zur Verbreiterung des Wirkungsspektrums und zur Erzielung synergistischer Effekte können die Verbindungen der Formel I sowohl untereinander als auch mit Vertretern anderer herbizider oder wachstumsregulierender Wirkstoffgruppen gemischt und gemeinsam ausgebracht werden. Beispielsweise kommen als Mischungspartner Diazin, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate, Chinolincarbonsäurederivate, Sulfonylharnstoffderivate, (Hetero)-aryloxy-phenoxypropionsäuren, deren Salze, Ester und Amide und andere in Betracht.

Außerdem kann es von Nutzen sein, die Verbindungen allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

Die folgenden Beispiele erläutern die Herstellung der Verbindungen Ia und Ib und deren Vorprodukte II bis V.

### Herstellung der Vorprodukte:

### Beispiel 1

### Thiophen-3,4-dicarbonsäureanhydrid

Thiophen-3,4-dicarbonsäure (12 g, 0,07 mol) wird in Acetanhydrid (60 ml) gelöst und 2,5 Stunden zum Sieden erhitzt. Anschließend wird zur Trockene eingeengt. Man erhält 10,55 g (98 %) Thiophen-3,4-dicarbonsäureanhydrid (Fp. 147 bis 149°C).

### Beispiel 2

### 4-(4-Chlorphenyl)aminocarbonyl-thiophen-3-carbonsäure

Thiophen-3,4-dicarbonsäureanhydrid (2 g, 0,013 mol) wird in 60 ml Toluol vorgelegt und mit 4-Chloranilin (1,65 g, 0,013 mol) versetzt. Man läßt 3 Stunden bei Raumtemperatur rühren, filtriert die ausgefallene Carbonsäure ab und wäscht mit wenig Toluol nach. Nach Trocknen erhält man 3,7 g (100 %) des gewünschten Produktes, Fp. 204 bis 208°C.

### Beispiel 3

### 4-(4-Chlorphenyl)aminocarbonyl-thiophen-3-carbonsäure-hydroxysuccinimidester

4-(4-Chlorphenyl)aminocarbonyl-thiophen-3-carbonsäure (1,7 g 0,006 mol) wird in 60 ml THF gelöst, mit N-Hydroxysuccinimid (0,7 g 0,006 mol) und N,N-Dicyclohexylcarbodiimid (1,25 g, 0,006 mol) versetzt und mehrere Stunden bei Raumtemperatur gerührt. Anschließend kühlt man die Lösung für einige Stunden auf 0°C, saugt den ausgefallenen Harnstoff ab und engt zur Trockene ein. Ausbeute: 1,8 g, 66 %; Fp. 65 bis 67°C.

### Beispiel 4

### 4-Chlorthiophen-3-carbonsäureanilid

4-Chlorthiophen-3-carbonsäurechlorid (13,5 g 0,075 mol) werden in 30 ml Dioxan gelöst und bei Raumtemperatur zu einer Lösung aus Anilin (7,7 g 0,083 mol) in Pyridin (160 ml) getropft. Man läßt 12 Stunden bei Raumtemperatur rühren, engt dann zur Trockene ein und nimmt den Rückstand in Dichlormethan auf. Die organische Phase wird mit wäßriger Citronensäurelösung, Natriumhydrogencarbonatlösung und Wasser extrahiert, getrocknet und zur Trockne eingeengt. Ausbeute: 16,7 g, 94 %; Fp. 119 bis 121°C.

### Beispiel 5

### 4-Chlor-3-phenylaminocarbonyl-thiophen-2-carbonsäure

4-Chlorthiophen-3-carbonsäureanilid (14,6 g, 0,061 mol) wird in 450 ml THF gelöst, auf -70°C gekühlt und mit n-Butyllithium (0,13 mol, 1,6 N Lösung in n-Hexan) versetzt. Nach 30 Minuten gast man bis zur Sättigung der Lösung Kohlendioxid ein und läßt langsam auf Raumtemperatur aufwärmen. Zur Aufarbeitung ent man bis zur Trockene ein, nimmt den festen Rückstand in einem Gemisch aus Wasser, Natronlauge und Ethylacetat auf, trennt die Phase und säuert die wäßrige Phase mit Salzsäure an. Dabei fällt die Carbonsäure aus. Ausbeute: 13,5 g; 78 %; Fp. 208 bis 210°C.

Nach den beschriebenen Methoden wurden z.B. die in den Tabellen a bis c dargestellten Dicarbonsäuremonoamide hergestellt.

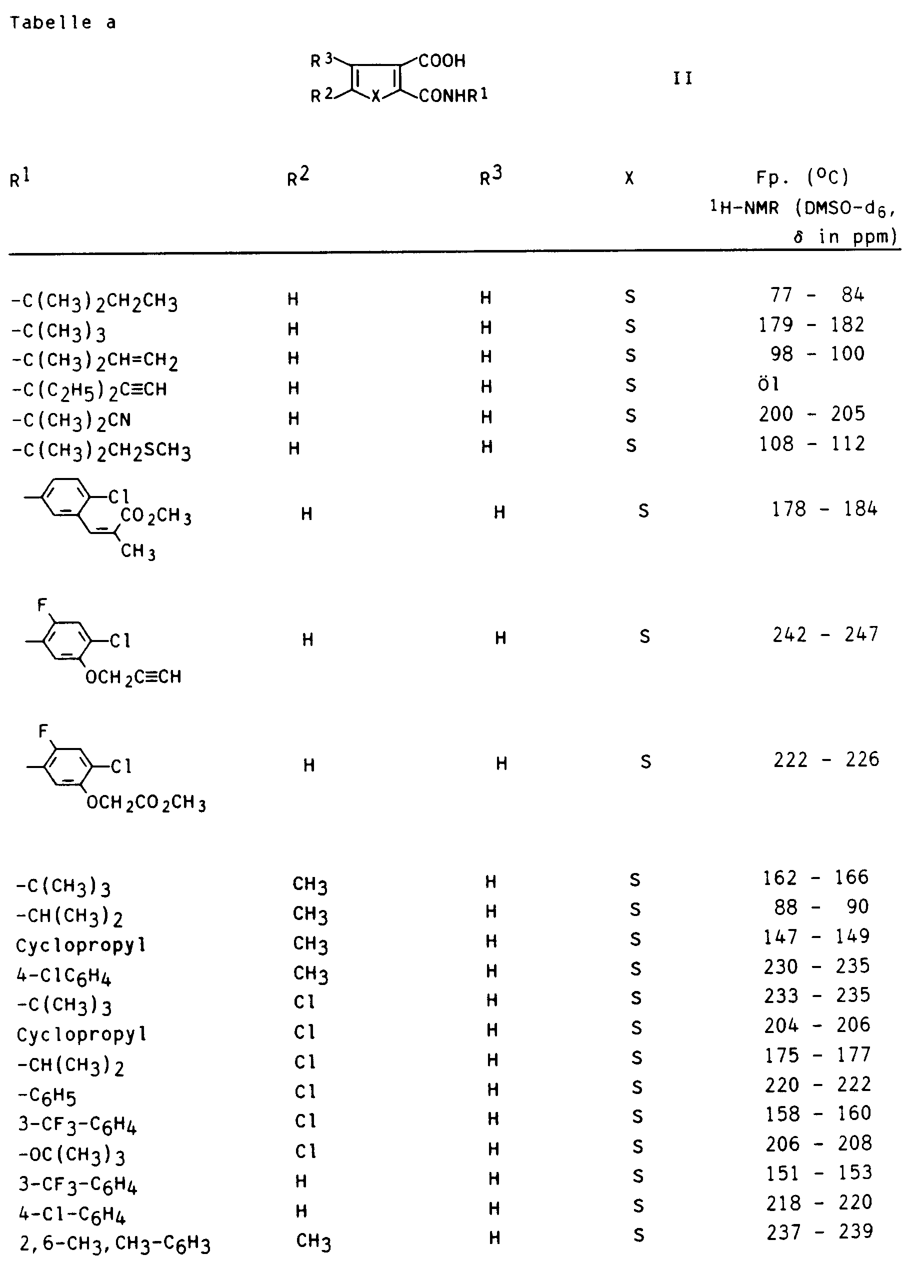

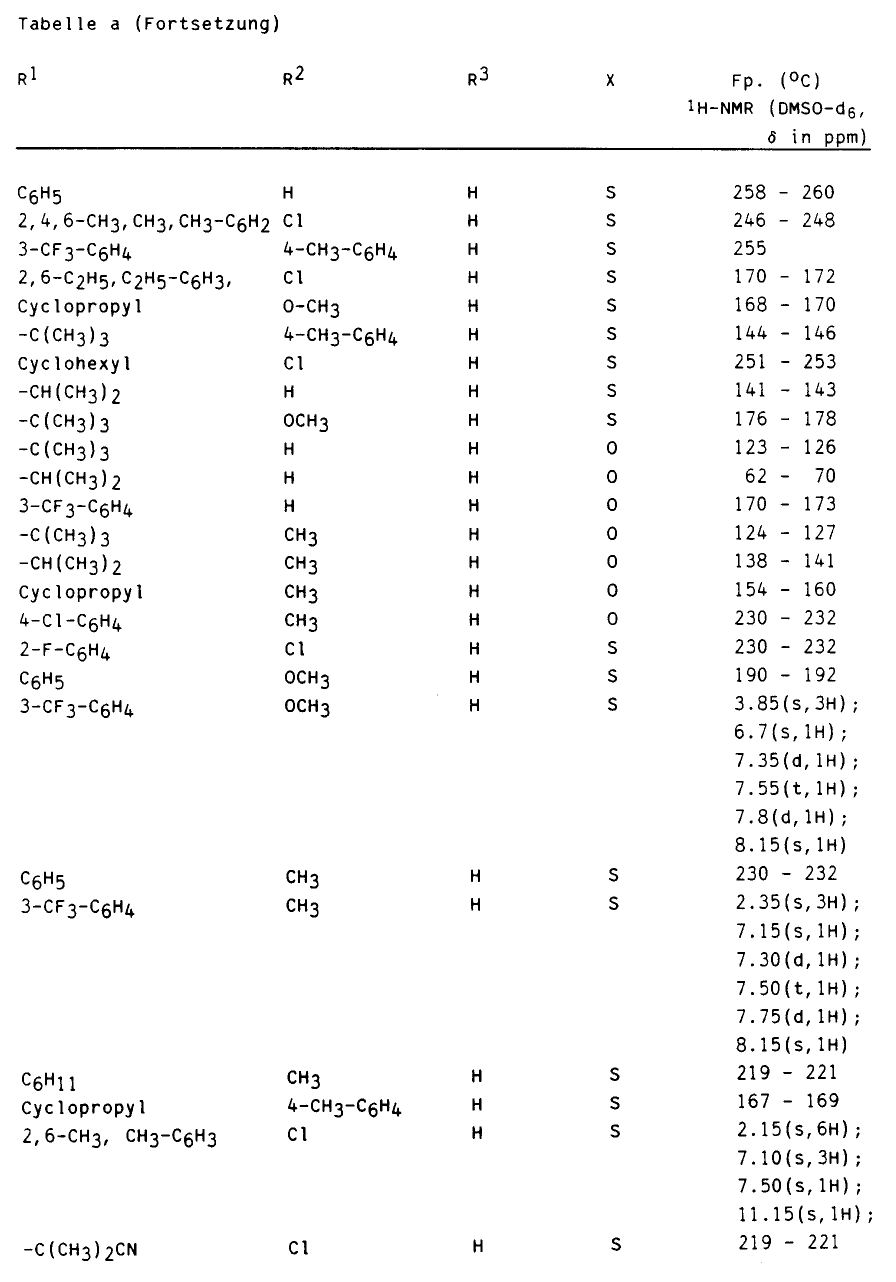

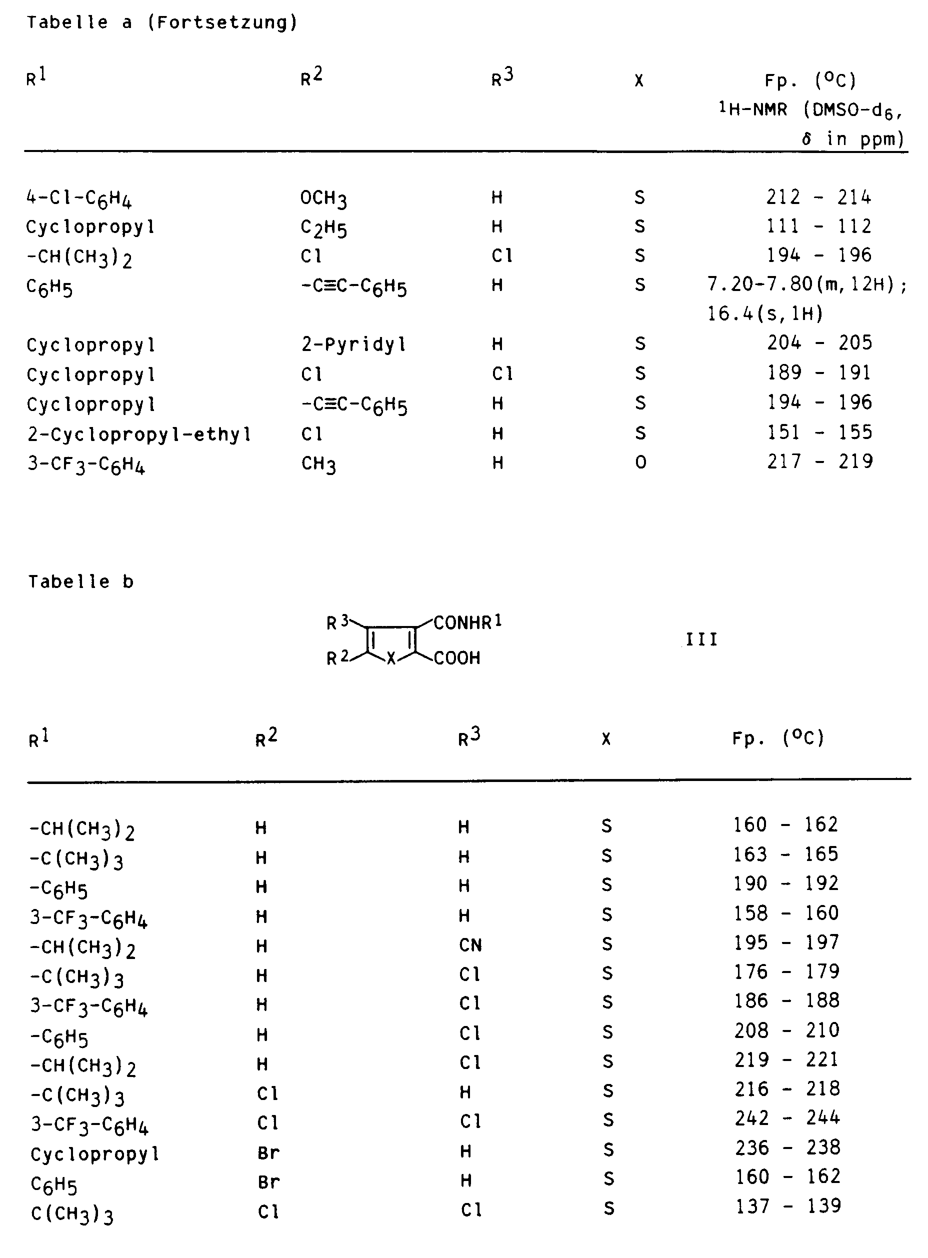

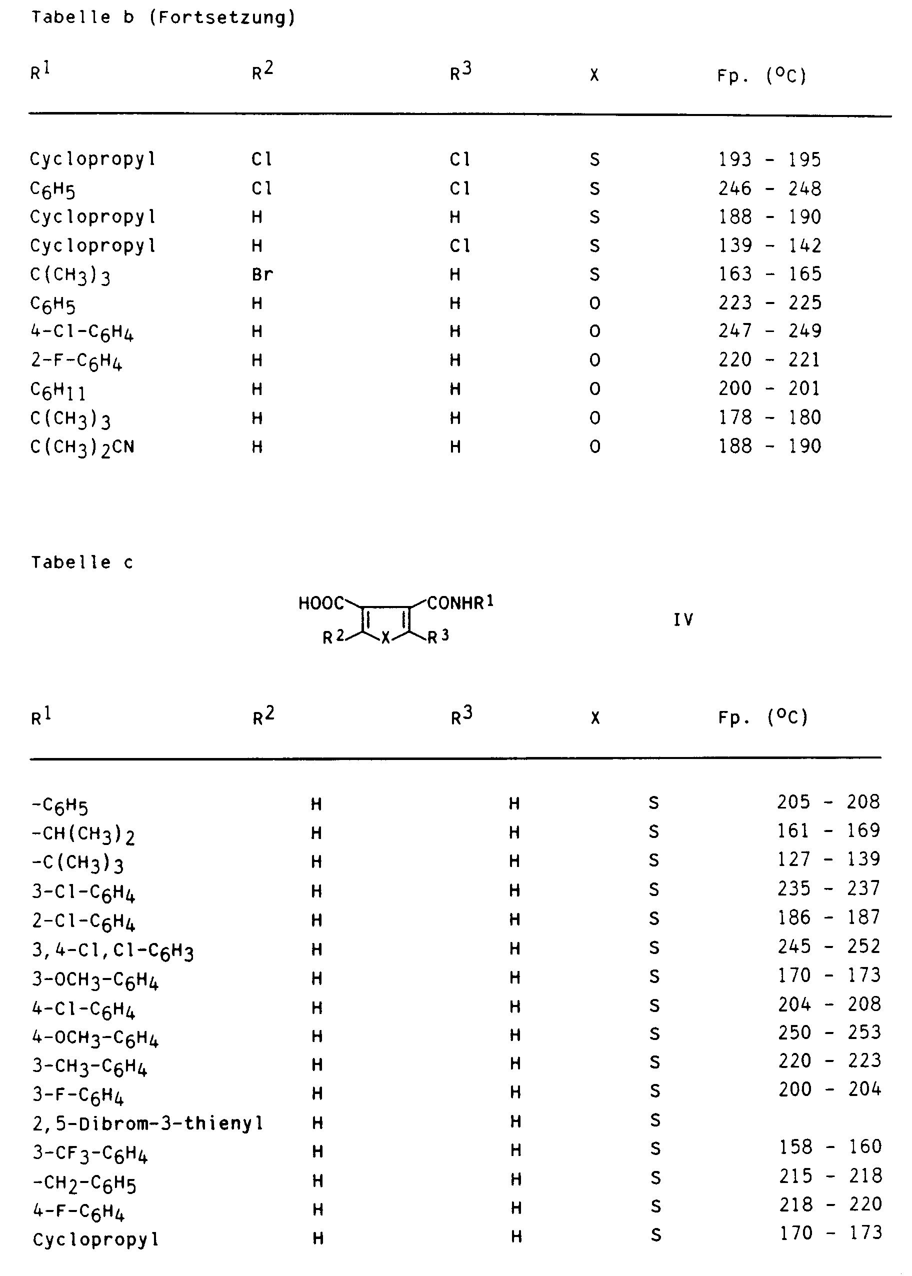

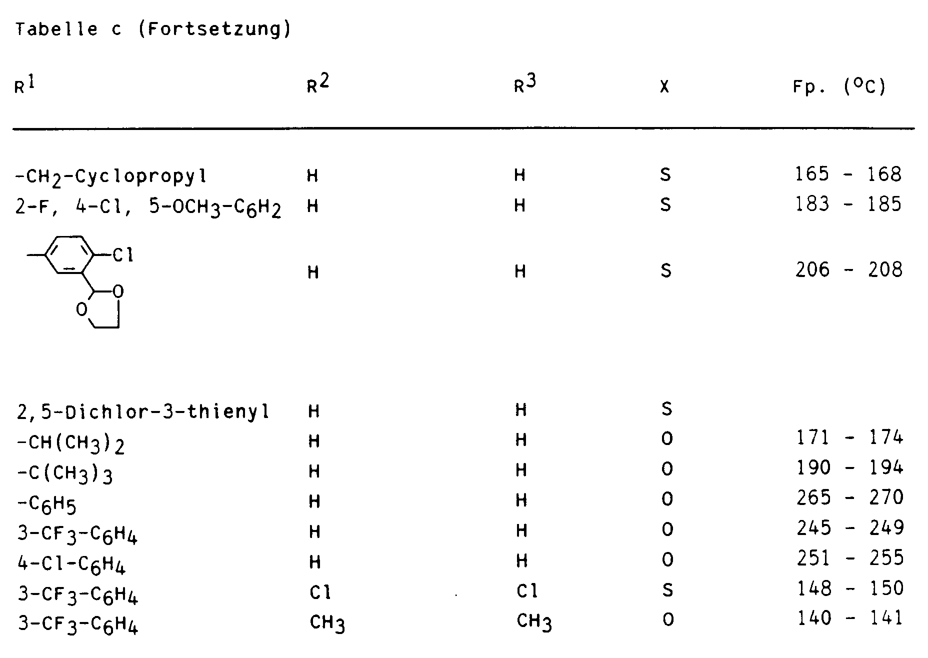

### Herstellung der Endprodukte Ia, Ib

### Beispiel 6

### N-Isopropyl-thiophen-3,4-carboximid

4-Isopropylaminocarbonyl-thiophen-3-carbonsäure (3 g, 0,014 mol) und Thionylchlorid (4,6 g, 0,064 mol) werden in 1,2-Dichlorethan 5 h zum Rückflluß erhitzt. Anschließend wird zur Trockene eingeengt und der Rückstand chromatographiert. Ausbeute 2,4 g 88 %, Fp. 127 - 129°C.

### Beispiel 7

### N-Phenyl-5-chlor-thiophen-2,3-carboximid

5-Chlor-2-phenylaminocarbonyl-thiophen-3-carbonsäure (1,1 g, 0,0039 mol) und Thionylchlorid (4 ml, 0,055 mol) werden in 1,2-Dichlorethan 5 h zum Rückfluß erhitzt. Anschließend wird zur Trockene eingeengt und der Rückstand chromatographiert. Ausbeute: 0,5 g; 49 %; Fp.: 176 - 178°.

### Beispiel 8

### N-tert-Butyl-5-methyl-furan-2,3-carboximid

2-tert-Butylaminocarbonyl-5-methyl-furan-3-carbonsäure (6,4 g, 0,028 mol) und p-Toluolsulfonsäure (6,5 g, 0,034 mol) werden 12 h bei Raumtemperatur in Pyridin gerührt. Anschließend wird zur Trockene eingeengt und der Rückstand chromatographiert. Ausbeute: 4,9 g, 98 %, Fp. 131 - 133°C.

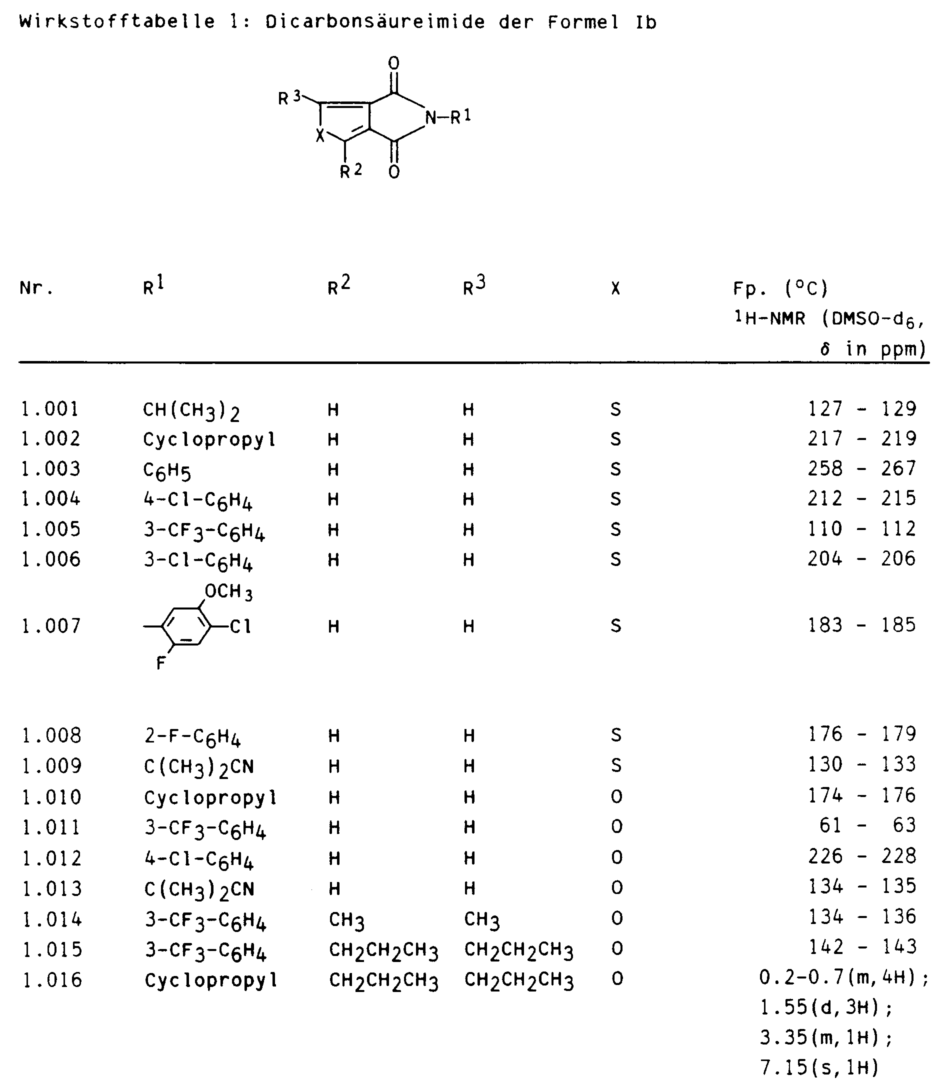

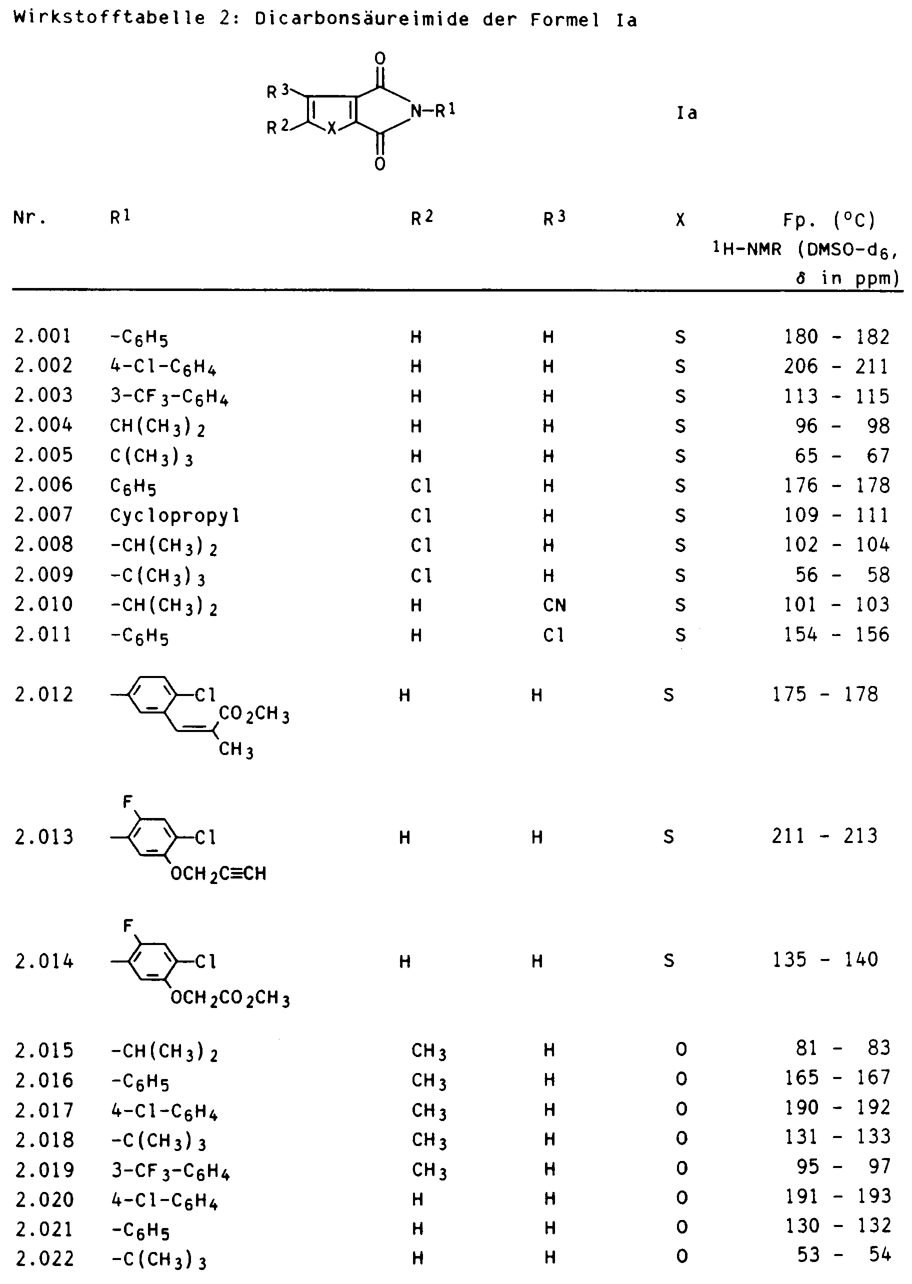

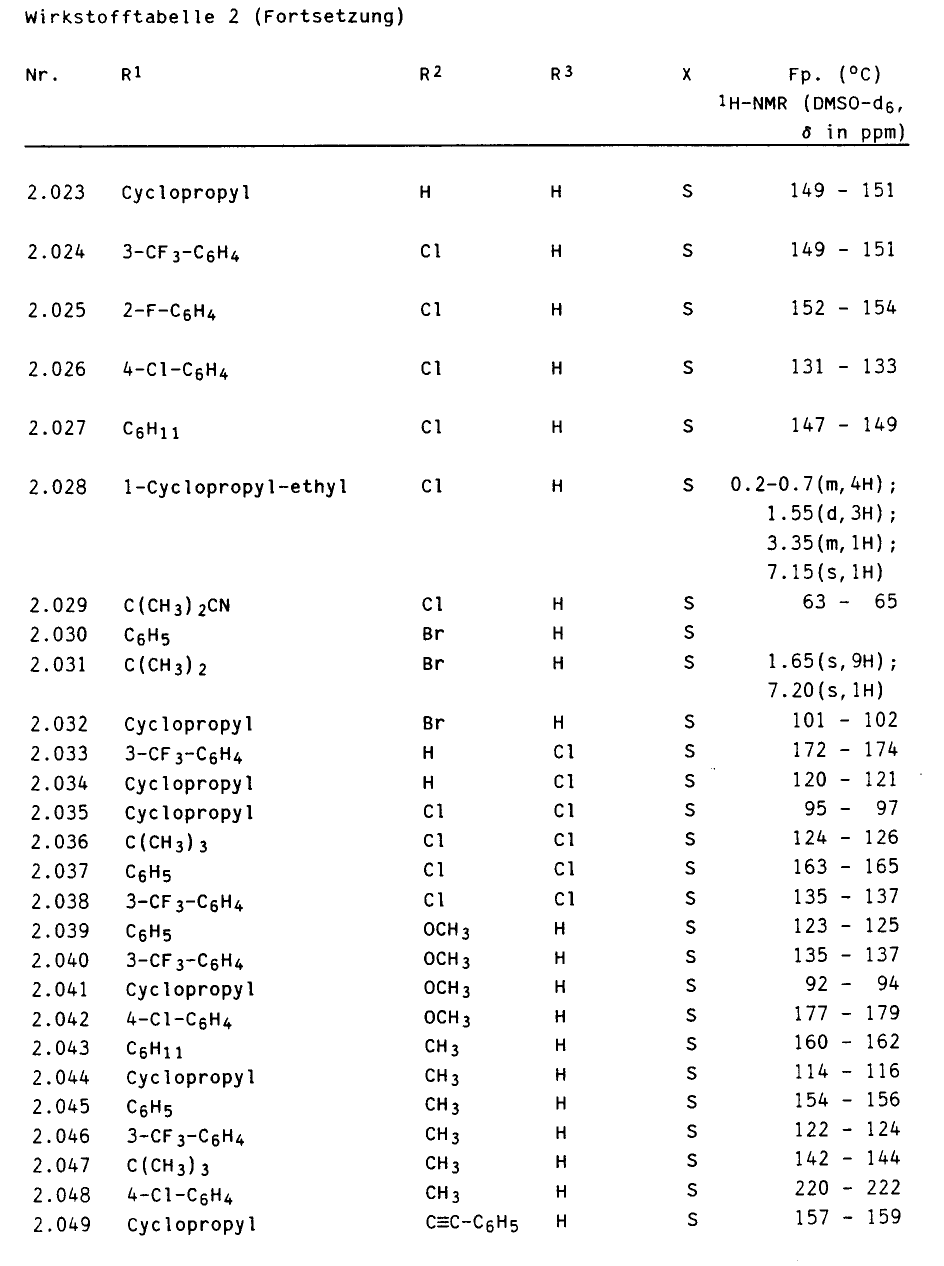

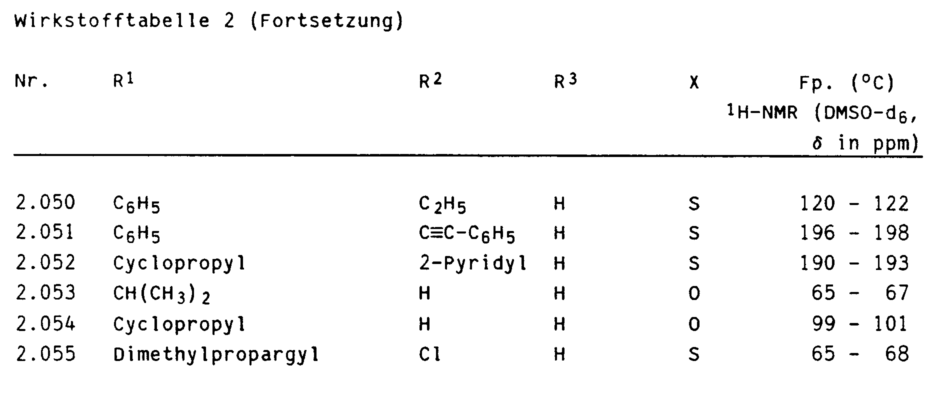

### Anwendungsbeispiele

Die herbizide Wirkung der Dicarbonsäureimide der Formel I ließ sich durch Gewächshausversuche zeigen:

Als Kulturgefäße dienten Plastikblumentöpfe mit lehmigem Sand mit etwa 3,0 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zwecke der Nachauflaufbehandlung wurden die Testpflanzen je nach Wuchsform erst bei einer Wuchshöhe von 3 bis 15 cm mit den in Wasser suspendierten oder emulgierten Wirkstoffen behandelt. Die Aufwandmenge für die Nachauflaufbehandlung betrug 1 kg/ha a.S.

Die Pflanzen wurden artenspezifisch bei Temperaturen von 10-25°C bzw. 20-35°C gehalten. Die Versuchsperiode erstreckte sich über 2 bis 4 Wochen. Während dieser Zeit wurden die Pflanzen gepflegt und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Lateinischer Name | Deutscher Name |
|---|---|
| Abutilon theophrasti | Chinesischer Hanf |
| Amaranthus retroflexus | Zurückgekrümmter Fuchsschwanz |
| Chenopodium album | Weißer Gänsefuß |
| Chrysanthemum coronarium | Kronenwucherblume |
| Solanum nigrum | Schwarzer Nachtschatten |

Mit 1 kg/ha a.S. im Nachauflaufverfahren eingesetzt, lassen sich mit den Beispielsverbindungen Nr. 1.005, 2.001 und 2.007 breitblättrige unerwünschte Pflanzen sehr gut bekämpfen, wobei die Wirkstoffe gleichzeitig gut verträglich sind für die Kulturpflanze Mais.

## Patentansprüche (Patentansprüche für folgende(n) Vertragsstaat(en): AT, BE, CH, DE, FR, GB, IT, LI, NL)

1. Dicarbonsäureimide der allgemeinen Formeln Ia und Ib, 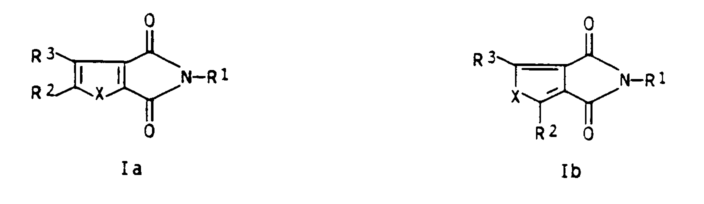 in denen die Substituenten folgende Bedeutung haben:
X
Sauerstoff oder Schwefel;
R¹
Wasserstoff, Hydroxy,
C₃-C₈-Cycloalkyl, welches ein bis drei der folgenden Reste tragen kann: Halogen, C₁-C₄-Alkyl, C₁-C₄-Halogenalkyl, C₁-C₄-Alkoxy und/oder C₁-C₄-Halogenalkoxy;
C₁-C₆-Alkyl, welches ein bis drei der folgenden Reste tragen kann: Hydroxy, Halogen, Cyano, C₃-C₈-Cycloalkyl, C₁-C₄-Alkoxy, C₁-C₄-Halogenalkoxy, C₁-C₄-Alkylthio, C₁-C₄-Halogenalkylthio, C₁-C₄-Alkylamino, Di-C₁-C₄-alkylamino und/oder C₃-C₆-Cycloalkylamino und/oder einen Rest 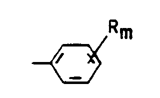 tragen kann, wobei
R
Cyano, Nitro, Halogen, C₁-C₄-Alkyl, C₁-C₄-Halogenalkyl, C₁-C₄-Alkoxy, C₁-C₄-Halogenalkoxy, C₂-C₄-Alkinyloxy, C₁-C₄-Alkylthio, C₁-C₄-Halogenalkylthio, C₃-C₆-Alkoxycarbonylalkoxy, C₁-C₄-Alkoxycarbonyl, 2-Alkoxycarbonylprop-1-enyl, C₁-C₄-Alkanoyl, C₁-C₄-Halogenalkanoyl, Formyl, 2-Dioxolanyl und/oder Phenyl bedeutet und
m
für 0, 1, 2 oder 3 steht, wobei die Reste R verschieden sein können, wenn m 2 oder 3 bedeutet;
C₁-C₄-Alkoxy, C₂-C₆-Cyanalkyl, C₃-C₆-Alkenyl, C₃-C₆-Alkinyl, Phenyl oder Naphthyl, wobei diese Gruppen ein bis drei der bei R genannten Reste tragen können;
einen 5- bis 6-gliedrigen gesättigten oder ungesättigten Heterocyclus, enthaltend ein oder zwei Heteroatome ausgewählt aus der Gruppe Stickstoff, Sauerstoff und Schwefel, wobei dieser Ring ein oder zwei der folgenden Reste tragen kann: Halogen, C₁-C₄-Alkyl, C₁-C₄-Halogenalkyl, C₁-C₄-Alkoxy, C₁-C₄-Halogenalkoxy und/oder C₁-C₄-Alkylthio,
Di-C₁-C₄-alkylamino;
R², R³
Nitro, Cyano, Halogen,
Amino, welches ein oder zwei C₁-C₄-Alkylgruppen und/oder eine C₁-C₄-Alkylcarbonylgruppe tragen kann;
C₁-C₄-Alkoxy oder C₁-C₄-Alkylthio, wobei diese Gruppen ein bis neun Halogenatome tragen können;
C₁-C₄-Alkyl- oder -Halogenalkylsulfonyl
C₂-C₆-Alkenyl, C₂-C₆-Alkinyl, Phenyl, Phenoxy oder Phenylthio, wobei diese Gruppen ein bis drei der bei R genannten Reste tragen können, oder
eine der bei R¹ genannten Gruppen;
mit der Maßgabe, daß im Falle des Dicarbonsäureimids Ia mit X = Schwefel
- R¹ nicht Wasserstoff und 2-Methyl-4-nitro-phenyl bedeutet, wenn R² und R³ für Wasserstoff stehen;
- R² nicht Amino bedeutet, wenn R³ für Cyano steht;
ferner mit der Maßgabe, daß im Fall des Dicarbonsäureimids Ib mit X = Sauerstoff
- R¹ nicht Phenyl oder Wasserstoff bedeutet, wenn R² und R³ für Phenyl stehen;
- nicht alle Reste R¹ - R³ gleichzeitig Methyl bedeuten;
sowie schließlich mit der Maßgabe, daß im Fall der Dicarbonsäureimide Ib mit X = Schwefel
- R¹ nicht Phenyl bedeutet, wenn R² und R³ für Wasserstoff stehen;
- nicht alle Reste R¹ - R³ gleichzeitig Wasserstoff bedeuten;
- R² und R³ nicht gleichzeitig für Phenyl, das ein Rest Chlor, Brom, Cyano, Alkoxycarbonyl oder Alkoxy tragen kann, stehen;
sowie landwirtschaftlich brauchbare Salze der Verbindungen Ia oder Ib.

2. Verfahren zur Herstellung der Verbindungen Ia gemäß Anspruch 1, dadurch gekennzeichnet, daß man ein Dicarbonsäuremonoamid der Formel II bzw. III 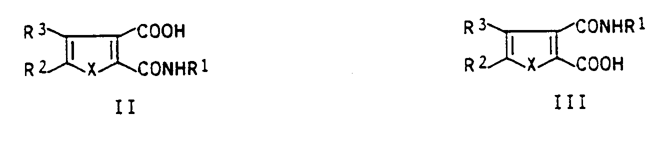 in an sich bekannter Weise mit wasserabspaltenden Mittlen umsetzt.

3. Verfahren zur Herstellung der Berbindungen Ib gemäß Anspruch 1, dadurch gekennzeichnet, daß man ein Dicarbonsäuremonoamid der Formel IV bzw. V 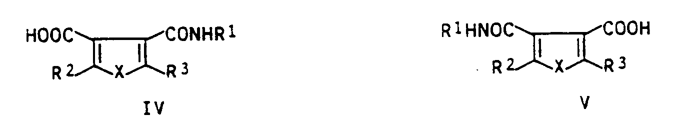 in an sich bekannter Weise mit wasserabspaltenden Mitteln umsetzt.

4. Verfahren zur Herstellung der Verbindungen Ia, Ib gemäß Anspruch 1, dadurch gekennzeichnet, daß man ein Dicarbonsäureanhydrid der Formel VI bzw. VII 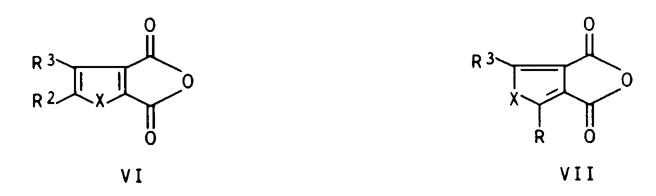 in an sich bekannter Weise mit einem Amin H₂NR¹ (VIII) umsetzt.

5. Herbizide Mittel, enthaltend neben inerten Zusatzstoffen mindestens ein Dicarbonsäureimid der allgemeinen Formeln Ia oder Ib 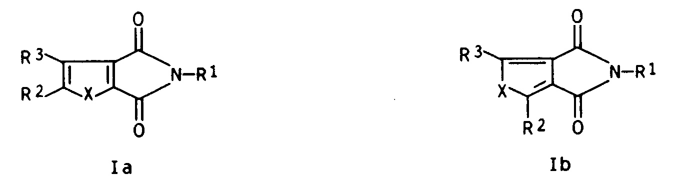 in denen die Substituenten folgende Bedeutung haben:
X
Sauerstoff oder Schwefel;
R¹
Wasserstoff, Hydroxy
C₃-C₈-Cycloalkyl, welches ein bis drei der folgenden Reste tragen kann: Halogen, C₁-C₄-Alkyl, C₁-C₄-Halogenalkyl, C₁-C₄-Alkoxy und/oder C₁-C₄-Halogenalkoxy;
C₁-C₆-Alkyl, welches ein bis drei der folgenden Reste tragen kann: Hydroxy, Halogen, Cyano, C₃-C₈-Cycloalkyl, C₁-C₄-Alkoxy, C₁-C₄-Halogenalkoxy, C₁-C₄-Alkylthio, C₁-C₄-Halogenalkylthio, C₁-C₄-Alkylamino, Di-C₁-C₄-alkylamino und/oder C₃-C₆-Cycloalkylamino und/oder einen Rest 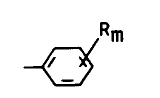 tragen kann, wobei
R
Cyano, Nitro, Halogen, C₁-C₄-Alkyl, C₁-C₄-Halogenalkyl, C₁-C₄-Alkoxy, C₁-C₄-Halogenalkoxy, C₂-C₄-Alkinyloxy, C₁-C₄-Alkylthio, C₁-C₄-Halogenalkylthio, C₃-C₆-Alkoxycarbonylalkoxy, C₁-C₄-Alkoxycarbonyl, 2-Alkoxycarbonylprop-1-enyl, C₁-C₄-Alkanoyl, C₁-C₄-Halogenalkanoyl, Formyl, 2-Dioxolanyl und/oder Phenyl bedeutet und
m
für 0, 1, 2 oder 3 steht, wobei die Reste R verschieden sein können, wenn m 2 oder 3 bedeutet;
C₁-C₄-Alkoxy, C₂-C₆-Cyanalkyl, C₃-C₆-Alkenyl, C₃-C₆-Alkinyl, Phenyl oder Naphthyl, wobei diese Gruppen ein bis drei der bei R genannten Reste tragen können;
einen 5- bis 6-gliedrigen gesättigten oder ungesättigten Heterocyclus, enthaltend ein oder zwei Heteroatome ausgewählt aus der Gruppe Stickstoff, Sauerstoff und Schwefel, wobei dieser Ring ein oder zwei der folgenden Reste tragen kann: Halogen, C₁-C₄-Alkyl, C₁-C₄-Halogenalkyl, C₁-C₄-Alkoxy, C₁-C₄-Halogenalkoxy und/oder C₁-C₄-Alkylthio,
Di-C₁-C₄-alkylamino;
R², R³
Nitro, Cyano, Halogen,
Amino, welches ein oder zwei C₁-C₄-Alkylgruppen und/oder eine C₁-C₄-Alkylcarbonylgruppe tragen kann;
C₁-C₄-Alkoxy oder C₁-C₄-Alkylthio, wobei diese Gruppen ein bis neun Halogenatome tragen können;
C₁-C₄-Alkyl- oder -Halogenalkylsulfonyl
C₂-C₆-Alkenyl, C₂-C₆-Alkinyl, Phenyl, Phenoxy oder Phenylthio, wobei diese Gruppen ein bis drei der bei R genannten Reste tragen können, oder
eine der bei R¹ genannten Gruppen.

6. Verfahren zur Bekämpfung unerwünschten Pflanzenwuchses, dadurch gekennzeichnet, daß man die unerwünschten Pflanzen und/oder ihren Lebensraum mit einer herbizid wirksamen Menge eines Dicarbonsäureimids Ia oder Ib gemäß Anspruch 1 behandelt.

## Patentansprüche (Patentansprüche für folgende(n) Vertragsstaat(en): ES)

1. Herbizide Mittel, enthaltend neben inerten Zusatzstoffen mindestens ein Dicarbonsäureimid der allgemeinen Formeln Ia oder Ib 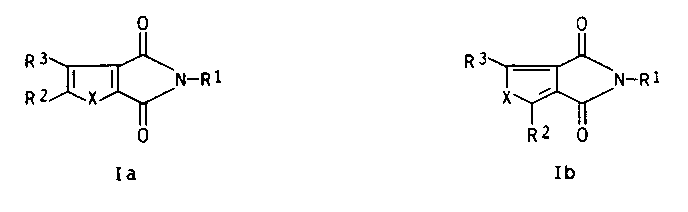 in denen die Substituenten folgende Bedeutung haben:
X
Sauerstoff oder Schwefel;
R¹
Wasserstoff, Hydroxy
C₃-C₈-Cycloalkyl, welches ein bis drei der folgenden Reste tragen kann: Halogen, C₁-C₄-Alkyl, C₁-C₄-Halogenalkyl, C₁-C₄-Alkoxy und/oder C₁-C₄-Halogenalkoxy;
C₁-C₆-Alkyl, welches ein bis drei der folgenden Reste tragen kann: Hydroxy, Halogen, Cyano, C₃-C₈-Cycloalkyl, C₁-C₄-Alkoxy, C₁-C₄-Halogenalkoxy, C₁-C₄-Alkylthio, C₁-C₄-Halogenalkylthio, C₁-C₄-Alkylamino, Di-C₁-C₄-alkylamino und/oder C₃-C₆-Cycloalkylamino und/oder einen Rest 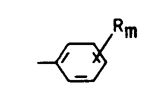 tragen kann, wobei
R
Cyano, Nitro, Halogen, C₁-C₄-Alkyl, C₁-C₄-Halogenalkyl, C₁-C₄-Alkoxy, C₁-C₄-Halogenalkoxy, C₂-C₄-Alkinyloxy, C₁-C₄-Alkylthio, C₁-C₄-Halogenalkylthio, C₃-C₆-Alkoxycarbonylalkoxy, C₁-C₄-Alkoxycarbonyl, 2-Alkoxycarbonylprop-1-enyl, C₁-C₄-Alkanoyl, C₁-C₄-Halogenalkanoyl, Formyl, 2-Dioxolanyl und/oder Phenyl bedeutet und
m
für 0, 1, 2 oder 3 steht, wobei die Reste R verschieden sein können, wenn m 2 oder 3 bedeutet;
C₁-C₄-Alkoxy, C₂-C₆-Cyanalkyl, C₃-C₆-Alkenyl, C₃-C₆-Alkinyl, Phenyl oder Naphthyl, wobei diese Gruppen ein bis drei der bei R genannten Reste tragen können;
einen 5- bis 6-gliedrigen gesättigten oder ungesättigten Heterocyclus, enthaltend ein oder zwei Heteroatome ausgewählt aus der Gruppe Stickstoff, Sauerstoff und Schwefel, wobei dieser Ring ein oder zwei der folgenden Reste tragen kann: Halogen, C₁-C₄-Alkyl, C₁-C₄-Halogenalkyl, C₁-C₄-Alkoxy, C₁-C₄-Halogenalkoxy und/oder C₁-C₄-Alkylthio,
Di-C₁-C₄-alkylamino;
R², R³
Nitro, Cyano, Halogen,
Amino, welches ein oder zwei C₁-C₄-Alkylgruppen und/oder eine C₁-C₄-Alkylcarbonylgruppe tragen kann;
C₁-C₄-Alkoxy oder C₁-C₄-Alkylthio, wobei diese Gruppen ein bis neun Halogenatome tragen können;
C₁-C₄-Alkyl- oder -Halogenalkylsulfonyl
C₂-C₆-Alkenyl, C₂-C₆-Alkinyl, Phenyl, Phenoxy oder Phenylthio, wobei diese Gruppen ein bis drei der bei R genannten Reste tragen können, oder
eine der bei R¹ genannten Gruppen;
mit der Maßgabe, daß im Falle des Dicarbonsäureimids Ia mit X = Schwefel
- R¹ nicht Wasserstoff und 2-Methyl-4-nitro-pheny bedeutet, wenn R² und R³ für Wasserstoff stehen;
- R² nicht Amino bedeutet, wenn R³ für Cyano steht;
ferner mit der Maßgabe, daß im Fall des Dicarbonsäureimids Ib mit X = Sauerstoff
- R¹ nicht Phenyl oder Wasserstoff bedeutet, wenn R² und R³ für Phenyl stehen;
- nicht alle Reste R¹ - R³ gleichzeitig Methyl bedeuten;
sowie schließlich mit der Maßgabe, daß im Fall der Dicarbonsäureimide Ib mit X = Schwefel
- R¹ nicht Phenyl bedeutet, wenn R² und R³ für Wasserstoff stehen;
- nicht alle Reste R¹ - R³ gleichzeitig Wasserstoff bedeuten;
- R² und R³ nicht gleichzeitig für Phenyl, das ein Rest Chlor, Brom, Cyano, Alkoxycarbonyl oder Alkoxy tragen kann, stehen;
sowie landwirtschaftlich brauchbare Salze der Verbindungen Ia oder Ib.

2. Verfahren zur Herstellung der Verbindungen Ia gemäß Anspruch 1, dadurch gekennzeichnet, daß man ein Dicarbonsäuremonoamid der Formel II bzw. III 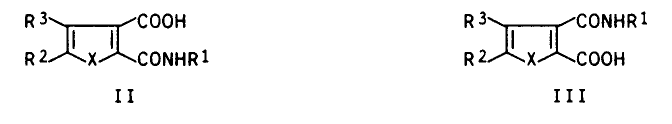 in an sich bekannter Weise mit wasserabspaltenden Mitteln umsetzt.

3. Verfahren zur Herstellung der Verbindungen Ib gemäß Anspruch 1, dadurch gekennzeichnet, daß man ein Dicarbonsäuremonoamid der Formel IV bzw. V 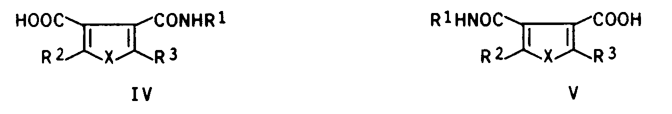 in an sich bekannter Weise mit wasserabspaltenden Mitteln umsetzt.

4. Verfahren zur Herstellung der Verbindungen Ia, Ib gemäß Anspruch 1, dadurch gekennzeichnet, daß man ein Dicarbonsäureanhydrid der Formel VI bzw. VII 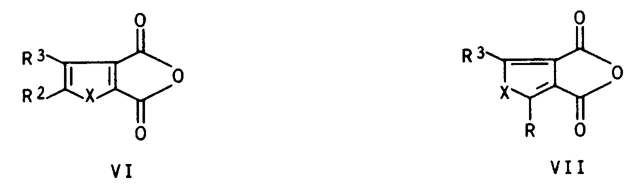 in an sich bekannter Weise mit einem Amin H₂NR¹ (VIII) umsetzt.

5. Verfahren zur Bekämpfung unerwünschten Pflanzenwuchses, dadurch gekennzeichnet, daß man die unerwünschten Pflanzen und/oder ihren Lebensraum mit einer herbizid wirksamen Menge eines Dicarbonsäureimids Ia oder Ib gemäß Anspruch 1 behandelt.

## Claims (Claims for the following Contracting State(s): AT, BE, CH, DE, FR, GB, IT, LI, NL)

1. A dicarboximide of the formula Ia or Ib 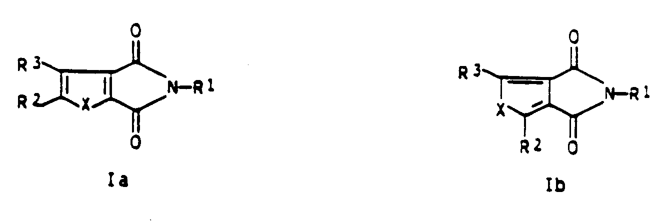 where
X is oxygen or sulfur;
R¹ is hydrogen or hydroxyl;
C₃-C₈-cycloalkyl which may carry from one to three of the following radicals: halogen, C₁-C₄-alkyl, C₁-C₄-haloalkyl, C₁-C₄-alkoxy and/or C₁-C₄-haloalkoxy;
C₁-C₆-alkyl which may carry from one to three of the following radicals: hydroxyl, halogen, cyano, C₃-C₈-cycloalkyl, C₁-C₄-alkoxy, C₁-C₄-haloalkoxy, C₁-C₄-alkylthio, C₁-C₄-haloalkylthio, C₁-C₄-alkylamino, di-C₁-C₄-alkylamino and/or C₃-C₆-cycloalkylamino and/or a radical 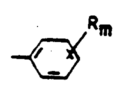 in which
R is cyano, nitro, halogen, C₁-C₄-alkyl, C₁-C₄-haloalkyl, C₁-C₄-alkoxy, C₁-C₄-haloalkoxy, C₂-C₄-alkynyloxy, C₁-C₄-alkylthio, C₁-C₄-haloalkylthio, C₃-C₆-alkoxycarbonylalkoxy, C₁-C₄-alkoxycarbonyl, 2-alkoxycarbonylprop 1-enyl, C₁-C₄-alkanoyl, C₁-C₄-haloalkanoyl, formyl, dioxolanyl and/or phenyl and
m is 0, 1, 2 or 3, and the radicals R may be different when m is 2 or 3;
C₁-C₄-alkoxy, C₂-C₆-cyanoalkyl, C₃-C₆-alkenyl, C₃-C₆-alkynyl, phenyl or naphthyl, where these groups may carry from one to three of the radicals stated for R;
a 5-membered or 6-membered saturated or unsaturated heterocyclic structure containing one or two hetero atoms selected from the group consisting of nitrogen, oxygen and sulfur, where this ring may carry one or two of the following radicals: halogen, C₁-C₄-alkyl, C₁-C₄-haloalkyl, C₁-C₄-alkoxy, C₁-C₄-haloalkoxy and/or C₁-C₄-alkylthio, or di-C₁-C₄-alkylamino;
R² and R³ are each nitro, cyano, halogen; amino which may carry one or two C₁-C₄-alkyl groups and/or a C₁-C₄-alkylcarbonyl group;
C₁-C₄-alkoxy or C₁-C₄-alkylthio, where these groups may carry from one to nine halogen atoms;
C₁-C₄-alkylsulfonyl or C₁-C₄-haloalkylsulfonyl;
C₂-C₆-alkenyl, C₂-C₆-alkynyl, phenyl, phenoxy or phenylthio, where these groups may carry from one to three of the radicals stated for R, or
one of the groups stated for R¹,
with the proviso that, in the case of the dicarboximide Ia where X is sulfur,
R¹ is not hydrogen or 2-methyl-4-nitrophenyl when R² and
R³ are each hydrogen and R² is not amino when R³ is cyano, and furthermore with the proviso that, in the case of the dicarboximide Ib where X is oxygen,
R¹ is not phenyl or hydrogen when R² and R³ are each phenyl and
all radicals R¹ - R³ are not simultaneously methyl, and finally with the proviso that, in the case of the dicarboximides Ib where X is sulfur,
R¹ is not phenyl when R² and R³ are each hydrogen,
all radicals R¹ - R³ are not simultaneously hydrogen and
R² and R³ are not simultaneously phenyl which may carry a chlorine, bromine, cyano, alkoxycarbonyl or alkoxy radical,
and agriculturally useable salts of the compounds Ia or Ib.

2. A process for the preparation of a compound Ia as claimed in claim 1, wherein a dicarboxylic acid monoamide of the formula II or III 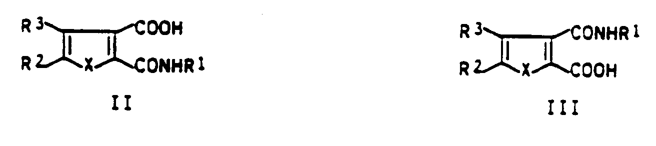 is reacted in a conventional manner with a water-eliminating agent.

3. A process for the preparation of a compound Ib as claimed in claim 1, wherein a dicarboxylic acid monoamide of the formula IV or V 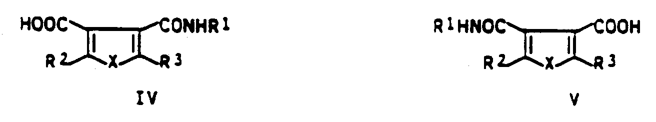 is reacted in a conventional manner with a water-eliminating agent.

4. A process for the preparation of a compound Ia or Ib as claimed in claim 1, wherein a dicarboxylic anhydride of the formula VI or VII 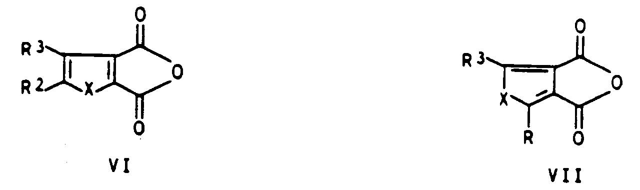 is reacted in a conventional manner with an amine H₂NR¹ (VIII).

5. A herbicide containing, in addition to inert additives, at least one dicarboximide of the formula Ia or Ib 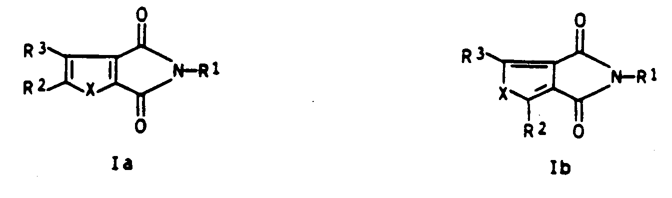 where
X is oxygen or sulfur;
R¹ is hydrogen or hydroxyl;
C₃-C₈-cycloalkyl which may carry from one to three of the following radicals: halogen, C₁-C₄-alkyl, C₁-C₄-haloalkyl, C₁-C₄-alkoxy and/or C₁-C₄-haloalkoxy;
C₁-C₆-alkyl which may carry from one to three of the following radicals: hydroxyl, halogen, cyano, C₃-C₈-cycloalkyl, C₁-C₄-alkoxy, C₁-C₄-haloalkoxy, C₁-C₄-alkylthio, C₁-C₄-haloalkylthio, C₁-C₄-alkylamino, di-C₁-C₄-alkylamino and/or C₃-C₆-cycloalkylamino and/or a radical 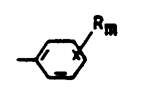 in which
R is cyano, nitro, halogen, C₁-C₄-alkyl, C₁-C₄-haloalkyl, C₁-C₄-alkoxy, C₁-C₄-haloalkoxy, C₂-C₄-alkynyloxy, C₁-C₄-alkylthio, C₁-C₄-haloalkylthio, C₃-C₆-alkoxycarbonylalkoxy, C₁-C₄-alkoxycarbonyl, 2-alkoxycarbonylprop-1-enyl, C₁-C₄-alkanoyl, C₁-C₄-haloalkanoyl, formyl, 2-dioxolanyl and/or phenyl and
m is 0, 1, 2 or 3, and the radicals R may be different when m is 2 or 3;
C₁-C₄-alkoxy, C₂-C₆-cyanoalkyl, C₃-C₆-alkenyl, C₃-C₆-alkynyl, phenyl or naphthyl, where these groups may carry from one to three of the radicals stated for R;
a 5-membered or 6-membered saturated or unsaturated heterocyclic structure containing one or two hetero atoms selected from the group consisting of nitrogen, oxygen and sulfur, where this ring may carry one or two of the following radicals: halogen, C₁-C₄-alkyl, C₁-C₄-haloalkyl, C₁-C₄-alkoxy, C₁-C₄-haloalkoxy and/or C₁-C₄-alkylthio, or di-C₁-C₄-alkylamino;
R² and R³ are each nitro, cyano, halogen; amino which may carry one or two C₁-C₄-alkyl groups and/or a C₁-C₄-alkylcarbonyl group;
C₁-C₄-alkoxy or C₁-C₄-alkylthio, where these groups may carry from one to nine halogen atoms;
C₁-C₄-alkylsulfonyl or C₁-C₄-haloalkylsulfonyl; C₂-C₆-alkenyl, C₂-C₆-alkynyl, phenyl, phenoxy or phenylthio, where these groups may carry from one to three of the radicals stated for R, or
one of the groups stated for R¹.

6. A method for controlling undesirable plant growth, wherein the undesired plants and/or their habitat are or is treated with a herbicidal amount of a dicarboximide Ia or Ib as claimed in claim 1.

## Claims (Claims for the following Contracting State(s): ES)

1. A herbicide containing, in addition to inert additives, at least one dicarboximide of the formula Ia or Ib 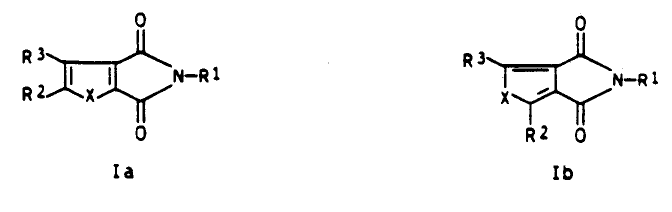 where
X is oxygen or sulfur;
R¹ is hydrogen or hydroxyl;
C₃-C₈-cycloalkyl which may carry from one to three of the following radicals: halogen, C₁-C₄-alkyl, C₁-C₄-haloalkyl, C₁-C₄-alkoxy and/or C₁-C₄-haloalkoxy;
C₁-C₆-alkyl which may carry from one to three of the following radicals: hydroxyl, halogen, cyano, C₃-C₈-cycloalkyl, C₁-C₄-alkoxy, C₁-C₄-haloalkoxy, C₁-C₄-alkylthio, C₁-C₄-haloalkylthio, C₁-C₄-alkylamino, di-C₁-C₄-alkylamino and/or C₃-C₆-cycloalkylamino and/or a radical 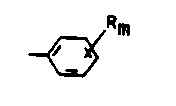 in which
R is cyano, nitro, halogen, C₁-C₄-alkyl, C₁-C₄-haloalkyl, C₁-C₄-alkoxy, C₁-C₄-haloalkoxy, C₂-C₄-alkynyloxy, C₁-C₄-alkylthio, C₁-C₄-haloalkylthio, C₃-C₆-alkoxycarbonylalkoxy, C₁-C₄-alkoxycarbonyl, 2-alkoxycarbonylprop-1-enyl, C₁-C₄-alkanoyl, C₁-C₄-haloalkanoyl, formyl, dioxolanyl and/or phenyl and
m is 0, 1, 2 or 3, and the radicals R may be different when m is 2 or 3;
C₁-C₄-alkoxy, C₂-C₆-cyanoalkyl, C₃-C₆-alkenyl, C₃-C₆-alkynyl, phenyl or naphthyl, where these groups may carry from one to three of the radicals stated for R;
a 5-membered or 6-membered saturated or unsaturated heterocyclic structure containing one or two hetero atoms selected from the group consisting of nitrogen, oxygen and sulfur, where this ring may carry one or two of the following radicals: halogen, C₁-C₄-alkyl, C₁-C₄-haloalkyl, C₁-C₄-alkoxy, C₁-C₄-haloalkoxy and/or C₁-C₄-alkylthio, or di-C₁-C₄-alkylamino;
R² and R³ are each nitro, cyano, halogen; amino which may carry one or two C₁-C₄-alkyl groups and/or a C₁-C₄-alkylcarbonyl group;
C₁-C₄-alkoxy or C₁-C₄-alkylthio, where these groups may carry from one to nine halogen atoms;
C₁-C₄-alkylsulfonyl or C₁-C₄-haloalkylsulfonyl;
C₂-C₆-alkenyl, C₂-C₆-alkynyl, phenyl, phenoxy or phenylthio, where these groups may carry from one to three of the radicals stated for R, or
one of the groups stated for R¹,
with the proviso that, in the case of the dicarboximide Ia where X is sulfur,
R¹ is not hydrogen or 2-methyl-4-nitrophenyl when R² and
R³ are each hydrogen and R² is not amino when R³ is cyano, and furthermore with the proviso that, in the case of the dicarboximide Ib where X is oxygen,
R¹ is not phenyl or hydrogen when R² and R³ are each phenyl and
all radicals R¹ - R³ are not simultaneously methyl, and finally with the proviso that, in the case of the dicarboximides Ib where X is sulfur,
R¹ is not phenyl when R² and R³ are each hydrogen, all radicals R¹ - R³ are not simultaneously hydrogen and
R² and R³ are not simultaneously phenyl which may carry a chlorine, bromine, cyano, alkoxycarbonyl or alkoxy radical,
and agriculturally useable salts of the compounds Ia or Ib.

2. A process for the preparation of a compound Ia as claimed in claim 1, wherein a dicarboxylic acid monoamide of the formula II or III 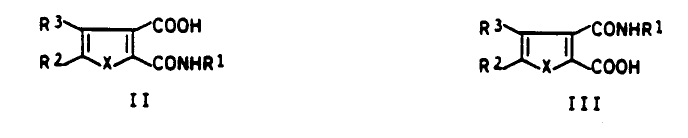 is reacted in a conventional manner with a water-eliminating agent.

3. A process for the preparation of a compound Ib as claimed in claim 1, wherein a dicarboxylic acid monoamide of the formula IV or V 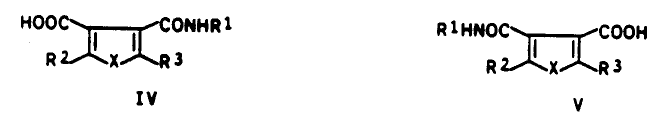 is reacted in a conventional manner with a water-eliminating agent.

4. A process for the preparation of a compound Ia or Ib as claimed in claim 1, wherein a dicarboxylic anhydride of the formula VI or VII 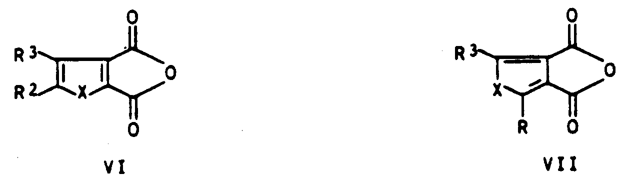 is reacted in a conventional manner with an amine H₂NR¹ (VIII).

5. A method for controlling undesirable plant growth, wherein the undesired plants and/or their habitat are or is treated with a herbicidal amount of a dicarboximide Ia or Ib as claimed in claim 1.

## Revendications (Revendications pour l'(les) Etat(s) contractant(s) suivant(s): AT, BE, CH, DE, FR, GB, IT, LI, NL)

1. Imides d'acides dicarboxyliques répondant aux formules générales Ia et Ib, 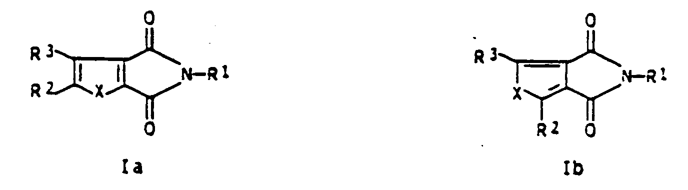 dans lesquelles les symboles ont les significations suivantes:
X
l'oxygène ou le soufre;
R¹
l'hydrogène, un groupe hydroxy, un groupe cycloalkyle en C3-C8 qui peut porter un à trois des substituants suivants: halogéno, alkyle en C1-C4, halogénoalkyle en C1-C4, alcoxy en C1-C4 et/ou halogénoalcoxy en C1-C4;
un groupe alkyle en C1-C6 qui peut porter un à trois des substituants suivants: hydroxy, halogéno, cyano, cycloalkyle en C3-C8, alcoxy en C1-C4, halogénoalcoxy en C1-C4, alkylthio en C1-C4, halogénoalkylthio en C1-C4, alkylamino en C1-C4, di-(alkyle en C1-C4)amino et/ou cycloalkylamino en C3-C6 et/ou un groupe 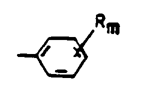 dans lequel
R représente
un groupe cyano, nitro, un halogène, un groupe alkyle en C1-C4, halogénoalkyle en C1-C4, alcoxy en C1-C4, halogénoalcoxy en C1-C4, alcynyloxy en C2-C4, alkylthio en C1-C4, halogénoalkylthio en C1-C4, alcoxycarbonylalcoxy en C3-C6, (alcoxy en C1-C4)carbonyle, 2-alcoxycarbonylpropa-1-ényle, alcanoyle en C1-C4, halogénoalcanoyle en C1-C4, formyle, 2-dioxolannyle et/ou phényle, et
m
est égal à 0, 1, 2 ou 3, les substituants R pouvant être différents lorsque m est égal à 2 ou 3;
un groupe alcoxy en C1-C4, cyanalkyle en C2-C6, alcényle en C3-C6, alcynyle en C3-C6, phényle ou naphtyle, ces groupes pouvant porter un à trois des substituants mentionnés en référence à R;
un hétérocycle saturé ou insaturé de cinq à six chaînons contenant un ou deux hétéro-atomes choisis parmi l'azote, l'oxygène et le soufre, ce cycle pouvant porter un ou deux des substituants suivants: halogéno, alkyle en C1-C4, halogénoalkyle en C1-C4, alcoxy en C1-C4, halogénoalcoxy en C1-C4 et/ou alkylthio en C1-C4,
un groupe di-(alkyle en C1-C4)amino;
R², R³
un groupe nitro, cyanao, un halogène,
un groupe amino qui peut porter un ou deux groupes alkyle en C1-C4 et/ou un groupe (alkyle en C1-C4)carbonyle;
un groupe alcoxy en C1-C4 ou alkylthio en C1-C4, ces groupes pouvant porter de un à neuf atomes d'halogènes;
un groupe (alkyle en C1-C4)- ou (halogénoalkyle en C1-C4)-sulfonyle,
un groupe alcényle en C2-C6, alcynyle en C2-C6, phényle, phénoxy ou phénylthio, ces groupes pouvant porter un à trois des substituants mentionnés en référence à R,
ou bien
l'un des groupes mentionnés en référence à R¹;
sous réserve que dans le cas de l'imide d'acide dicarboxylique Ia avec X = soufre
- R¹ ne peut représenter l'hydrogène ni un groupe 2-méthyl-4-nitrophényle lorsque R² et R³ représentent l'hydrogène;
- R² ne peut représenter un groupe amino lorsque R³ représente un groupe cyano;
et sous réserve également que dans le cas de l'imide d'acide dicarboxylique Ib avec X = oxygène,
- R¹ ne peut représenter un groupe phényle ni l'hydrogène lorsque R² et R³ représentent des groupes phényle;
- les symboles R¹ à R³ ne peuvent représenter tous simultanément, des groupes méthyle;
et finalement, sous réserve que dans le cas des imides d'acides dicarboxyliques Ib avec X = soufre,
- R¹ ne peut représenter un groupe phényle lorsque R² et R³ représentent l'hydrogène;
- les symboles R¹ à R³ ne peuvent représenter tous simultanément, l'hydrogène;
- R² et R³ ne peuvent représenter simultanément des groupes phényle pouvant porter un substituant chloro, bromo, cyano, alcoxycarbonyle ou alcoxy;
ainsi que les sels des composés Ia ou Ib aptes à des applications dans l'agriculture.

2. Procédé de préparation des composés Ia de la revendication 1, caractérisé par le fait que l'on fait réagir de manière connue en soi un monoamide d'acide dicarboxylique de formule respective II ou III 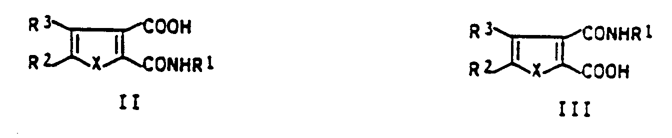 avec des agents provoquant la scission d'eau.

3. Procédé de préparation des composés Ib de la revendication 1, caractérisé par le fait que l'on fait réagir de manière connue en soi un monoamide d'acide dicarboxylique de formule respective IV ou V 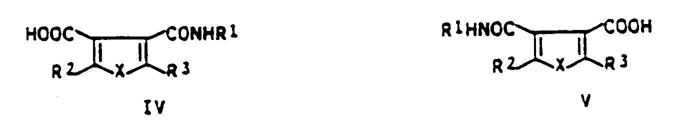 avec des agents provoquant la scission d'eau.

4. Procédé de préparation des composés Ia, Ib de la revendication 1, caractérisé par le fait que l'on fait réagir de manière connue en soi un anhydride d'acide dicarboxylique de formule VI ou VII 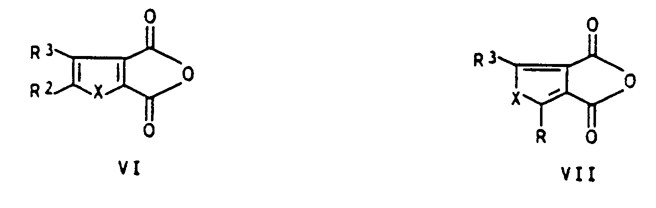 avec une amine H₂NR¹ (VIII).

5. Produit herbicide contenant, avec des additifs inertes, au moins un imide d'acide dicarboxylique de formule générale Ia ou Ib 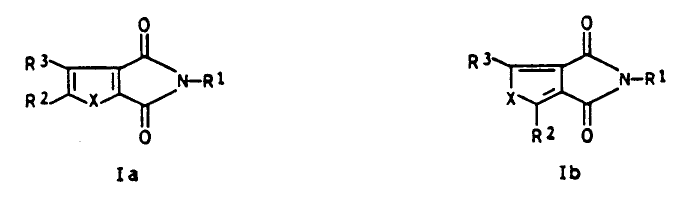 dans lesquelles les symboles ont les significations suivantes:
X
l'oxygène ou le soufre;
R¹
l'hydrogène, un groupe hydroxy,
un groupe cycloalkyle en C3-C8 qui peut porter un à trois des substituants suivants: halogéno, alkyle en C1-C4, halogénoalkyle en C1-C4, alcoxy en C1-C4 et/ou halogénoalcoxy en C1-C4;
un groupe alkyle en C1-C6 qui peut porter un à trois des substituants suivants: hydroxy, halogéno, cyano, cycloalkyle en C3-C8, alcoxy en C1-C4, halogénoalcoxy en C1-C4, alkylthio en C1-C4, halogénoalkylthio en C1-C4, alkylamino en C1-C4, di-(alkyle en C1-C4)amino et/ou cycloalkylamino en C3-C6 et/ou un groupe 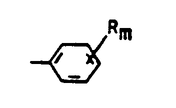 dans lequel
R représente
un groupe cyano, nitro, un halogène, un groupe alkyle en C1-C4, halogénoalkyle en C1-C4, alcoxy en C1-C4, halogénoalcoxy en C1-C4, alcynyloxy en C2-C4, alkylthio en C1-C4, halogénoalkylthio en C1-C4, alcoxycarbonylalcoxy en C3-C6, (alcoxy en C1-C4)carbonyle, 2-alcoxycarbonylpropa-1-ényle, alcanoyle en C1-C4, halogénoalcanoyle en C1-C4, formyle, 2-dioxolannyle et/ou phényle, et
m
est égal à 0, 1, 2 ou 3, les substituants R pouvant être différents lorsque m est égal à 2 ou 3;
un groupe alcoxy en C1-C4, cyanalkyle en C2-C6, alcényle en C3-C6, alcynyle en C3-C6, phényle ou naphtyle, ces groupes pouvant porter un à trois des substituants mentionnés en référence à R;
un hétérocycle saturé ou insaturé de cinq à six chaînons contenant un ou deux hétéroatomes choisis parmi l'azote, l'oxygène et le soufre, ce cycle pouvant porter un ou deux des substituants suivants: halogéno, alkyle en C1-C4, halogénoalkyle en C1-C4, alcoxy en C1-C4, halogénoalcoxy en C1-C4 et/ou alkylthio en C1-C4,
un groupe di-(alkyle en C1-C4)amino;
R², R³
un groupe nitro, cyano, un halogène,
un groupe amino qui peut porter un ou deux groupes alkyle en C1-C4 et/ou un groupe (alkyle en C1-C4)carbonyle;
un groupe alcoxy en C1-C4 ou alkylthio en C1-C4, ces groupes pouvant porter un à neuf atomes d'halogènes;
un groupe (alkyle en C1-C4)- ou (halogénoalkyle en C1-C4)sulfonyle,
un groupe alcényle en C2-C6, alcynyle en C2-C6, phényle, phénoxy ou phénylthio, ces groupes pouvant porter un à trois des substituants mentionnés en référence à R,
ou bien
l'un des groupes mentionnés en référence à R¹.

6. Procédé pour combattre les croissances de végétaux indésirables, caractérisé par le fait que l'on traite les végétaux indésirables et/ou leur habitat par une quantité herbicide efficace d'un imide d'acide dicarboxylique Ia ou Ib selon la revendication 1.

## Revendications (Revendications pour l'(les) Etat(s) contractant(s) suivant(s): ES)

1. Produit herbicide contenant, avec des additifs inertes, au moins un imide d'acide dicarboxylique de formule générale Ia ou Ib 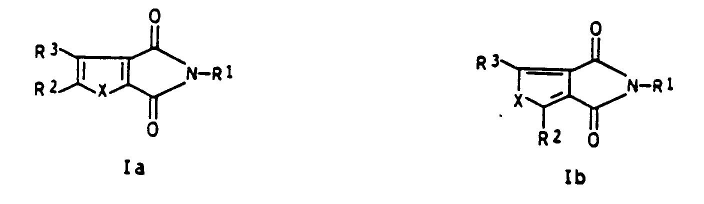 dans lesquelles les symboles ont les significations suivantes:
X
l'oxygène ou le soufre;
R¹
l'hydrogène, un groupe hydroxy,
un groupe cycloalkyle en C3-C8 qui peut porter un à trois des substituants suivants: halogéno, alkyle en C1-C4, halogénoalkyle en C1-C4, alcoxy en C1-C4 et/ou halogénoalcoxy en C1-C4;
un groupe alkyle en C1-C6 qui peut porter un à trois des substituants suivants: hydroxy, halogéno, cyano, cycloalkyle en C3-C8, alcoxy en C1-C4, halogénoalcoxy en C1-C4, alkylthio en C1-C4, halogénoalkylthio en C1-C4, alkylamino en C1-C4, di-(alkyle en C1-C4)amino et/ou cycloalkylamino en C3-C6 et/ou un groupe 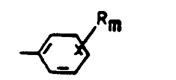 dans lequel
R représente
un groupe cyano, nitro, un halogène, un groupe alkyle en C1-C4, halogénoalkyle en C1-C4, alcoxy en C1-C4, halogénoalcoxy en C1-C4, alcynyloxy en C2-C4, alkylthio en C1-C4, halogénoalkylthio en C1-C4, alcoxycarbonylalcoxy en C3-C6, (alcoxy en C1-C4)carbonyle, 2-alcoxycarbonylpropa-1-ényle, alcanoyle en C1-C4, halogénoalcanoyle en C1-C4, formyle, 2-dioxolannyle et/ou phényle, et
m
est égal à 0, 1, 2 ou 3, les substituants R pouvant être différents lorsque m est égal à 2 ou 3;
un groupe alcoxy en C1-C4, cyanalkyle en C2-C6, alcényle en C3-C6, alcynyle en C3-C6, phényle ou naphtyle, ces groupes pouvant porter un à trois des substituants mentionnés en référence à R;
un hétérocycle saturé ou insaturé de cinq à six chaînons contenant un ou deux hétéro-atomes choisis parmi l'azote, l'oxygène et le soufre, ce cycle pouvant porter un ou deux des substituants suivants: halogéno, alkyle en C1-C4, halogénoalkyle en C1-C4, alcoxy en C1-C4, halogénoalcoxy en C1-C4 et/ou alkylthio en C1-C4,
un groupe di-(alkyle en C1-C4)amino;
R², R³
un groupe nitro, cyanao, un halogène,
un groupe amino qui peut porter un ou deux groupes alkyle en C1-C4 et/ou un groupe (alkyle en C1-C4)carbonyle;
un groupe alcoxy en C1-C4 ou alkylthio en C1-C4, ces groupes pouvant porter de un à neuf atomes d'halogènes;
un groupe (alkyle en C1-C4)- ou (halogénoalkyle en C1-C4)-sulfonyle,
un groupe alcényle en C2-C6, alcynyle en C2-C6, phényle, phénoxy ou phénylthio, ces groupes pouvant porter un à trois des substituants mentionnés en référence à R,
ou bien
l'un des groupes mentionnés en référence à R¹;
sous réserve que dans le cas de l'imide d'acide dicarboxylique Ia avec X = soufre
- R¹ ne peut représenter l'hydrogène ni un groupe 2-méthyl-4-nitrophényle lorsque R² et R³ représentent l'hydrogène;
- R² ne peut représenter un groupe amino lorsque R³ représente un groupe cyano;
et sous réserve également que dans le cas de l'imide d'acide dicarboxylique Ib avec X = oxygène,
- R¹ ne peut représenter un groupe phényle ni l'hydrogène lorsque R² et R³ représentent des groupes phényle;
- les symboles R¹ à R³ ne peuvent représenter tous simultanément, des groupes méthyle;
et finalement, sous réserve que dans le cas des imides d'acides dicarboxyliques Ib avec X = soufre,
- R¹ ne peut représenter un groupe phényle lorsque R² et R³ représentent l'hydrogène;
- les symboles R¹ à R³ ne peuvent représenter tous simultanément, l'hydrogène;
- R² et R³ ne peuvent représenter simultanément des groupes phényle pouvant porter un substituant chloro, bromo, cyano, alcoxycarbonyle ou alcoxy;
ainsi que les sels des composés Ia ou Ib aptes à des applications dans l'agriculture.

2. Procédé de préparation des composés Ia de la reendication 1, caractérisé par le fait que l'on fait réagir de manière connue en soi un monoamide d'acide dicarboxylique de formule II ou III 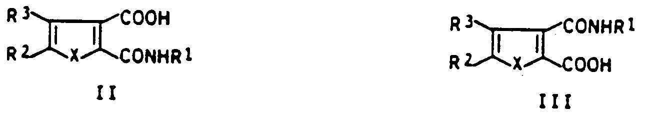 avec des agents provoquant la scission d'eau.

3. Procédé de préparation des composés Ib de la revendication 1, caractérisé par le fait que l'on fait réagir de manière connue en soi un monoamide d'acide dicarboxylique de formule IV ou V 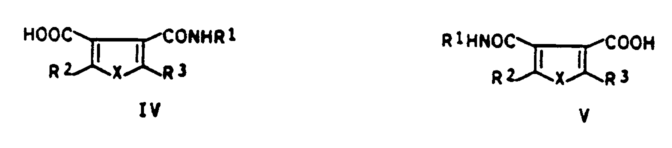 avec des agents provoquant la scission d'eau.

4. Procédé de préparation des composés Ia, Ib de la revendication 1, caractérisé par le fait que l'on fait réagir de manière connue en soi un anhydride d'acide dicarboxylique de formule respective VI ou VII 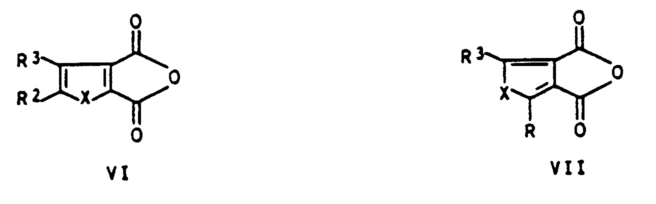 avec une amine H₂NR¹ (VIII).

5. Procédé pour combattre les croissances des végétaux indésirables, caractérisé par le fait que l'on traite les végétaux indésirables et/ou leur habitat par une quantité herbicide efficace d'un imide d'acide dicarboxylique Ia ou Ib selon la revendication 1.
